# DEMANDE DE BREVET EUROPEEN

(11) **EP 4 674 844 A1**
(43) Date de publication de la demande: **07.01.2026**
(21) Numéro de dépôt: 25186734.7
(22) Date de dépôt: 01.07.2025
(51) Int. Cl.: C07D 307/60, C07D 309/38, A01N 43/08, C07D 407/12

(54) **ANALOGUES DE LA CANNALACTONE, SYNTHESE ET UTILISATION POUR LA STIMULATION DE LA GERMINATION DE GRAINES DE PLANTES PARASITES**

(30) Priorité: 01.07.2024 FR 2407160
(71) Demandeur: Centre National de la Recherche Scientifique (CNRS), 75016 Paris (FR); Nantes Université, 44035 Nantes Cedex 1 (FR); Hemp it Adn, 49250 Beaufort-en-Anjou (FR)
(72) Inventeur: BOYER, François Didier, 78150 Le Chesnay-Rocquencourt (FR); POUVREAU, Jean Bernard, 44390 Nort sur Erdre (FR); DAIGNAN FORNIER DE LACHAUX, Suzanne, 91300 Massy (FR); MATHIS, Fabienne, 49100 Angers (FR)
(74) Mandataire: Oak & Fox

(57) **Abrégé**

La présente invention concerne la synthèse chimique d'analogues de la cannalactone, ainsi que l'utilisation de ces analogues pour la stimulation de la germination de graines de plantes parasites.

## Description

### Domaine technique de l'invention

La présente invention concerne la synthèse chimique d'analogues de la cannalactone, ainsi que l'utilisation de ces analogues pour la stimulation de la germination de graines de plantes parasites.

### Arrière-plan technique

Le chanvre (*Cannabis sativa*) est une plante annuelle originaire d'Asie exploitée depuis plus de 8 000 ans. Elle est cultivée dans le monde entier et est capable de couvrir les quatre besoins vitaux de l'humanité : se nourrir, se loger, s'habiller, se soigner.

La culture de chanvre est répandue car il s'agit d'une production rentable et durable. En effet, la plante s'adapte facilement aux terrains et aux climats divers et pour cela ne nécessite aucun traitement phytosanitaire. La surface de production de chanvre en France a été multipliée par trente entre 1960 et aujourd'hui. Cet intérêt pour le chanvre ne fait que croître, en partie dans le but de remplacer peu à peu le coton, très demandeur en eau. La France est aujourd'hui le premier producteur de chanvre en Europe avec une production sur environ 20 000 ha.

Connue pour sa résistance aux parasites et aux ravageurs, la plante de chanvre en est d'autant plus attractive. Cependant une plante parasite de la famille des orobanches, l'orobanche rameuse, *Phelipanche ramosa* induit de grandes pertes de rendement sur les cultures de chanvre pouvant aller jusqu'à plus de 80 %. Ce parasite s'attaque aussi à des cultures telles que le colza ou le tabac mais une spécialisation d'une population d'orobanche rameuse au chanvre a été mise en évidence [1]-[4].

Après germination, la plante parasite se connecte à la racine de la plante hôte et récupère ainsi les nutriments pour son propre développement [5]. Ce parasitisme crée des dommages importants sur les cultures de chanvre qui peuvent aller jusqu'à l'abandon de cette culture sur la parcelle.

Les strigolactones sont des petites molécules connues comme étant exsudées dans le sol à des concentrations picomolaires (10⁻¹² M) et ont été identifiées comme stimulants de germination des graines de *Phelipanche ramosa* ou orobanche rameuse [6], [7]. Les strigolactones (SL) ont été identifiées en premier pour leur rôle dans les interactions parasitaires [8] et symbiotiques [9] dans la rhizosphère et constituent la dernière classe d'hormones végétales à avoir été découverte [10], [11]. Elles sont surtout connues pour
leur rôle dans le contrôle de l'architecture des plantes, plus récemment, des rôles pour les SL dans d'autres aspects du développement des plantes ont été mise en évidence [12].

Pour disposer de molécules bioactives plus facilement, des analogues synthétiques des strigolactones ont été développés [4], [7], [13], [14] ont été développés. Les analogues sont des molécules de structure proche de celle des SL mais qui n'existent pas potentiellement dans la Nature.

Suite à la découverte de la cannalactone, la strigolactone découverte dans les exsudats de chanvre en quantité très faible et qui est le principal stimulant de germination de *P. ramosa*[1]-[4], le Demandeur a développé des analogues plus simples d'accès par rapport à la cannalactone et présentant une activité biologique dans certains cas supérieure à l'activité biologique de la cannalactone.

### Résumé de l'invention

En particulier, la présente invention a pour objet un analogue de la cannalactone, caractérisée en ce qu'il répond à la formule générale 1 : 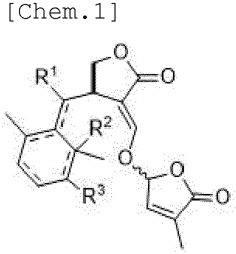 dans laquelle :
- R¹ désigne l'atome d'hydrogène H, le groupe hydroxyle OH ou le groupe OSiR⁴₃,
- R² et R³désignent chacun l'atome d'hydrogène H ou le radical méthyle CH₃,
- R⁴ désigne un groupement alkyle, et
- le cycle carboné à 6 chaînons pouvant être aromatique ou de type cyclohexène ou cyclohexane.

Selon un premier mode de réalisation de l'invention, l'analogue de la cannalactone selon l'invention peut être aromatique de stéréochimie « *cis* » et « *trans »* et répondant à la formule 2 : 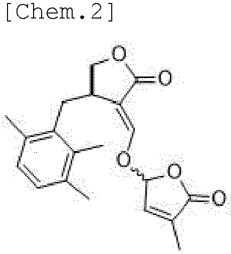 dans laquelle :
- R¹, R² et R³ désignent l'atome d'hydrogène H, et
- le cycle carboné à 6 chaînons est aromatique.

Selon un deuxième mode de réalisation de l'invention, l'analogue de la cannalactone selon l'invention peut être de type diène de stéréochimie « *cis* » et « *trans* » et répond à la formule 3 : 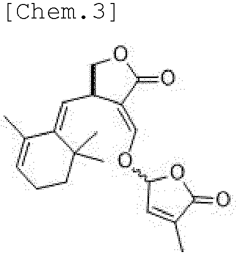 dans laquelle :
- R¹ et R³ désignent l'atome d'hydrogène H,
- R² désigne le groupement méthyle, et
- le cycle carboné à 6 chaînons est de type cyclohexène.

Selon un troisième mode de réalisation de l'invention, l'analogue de la cannalactone selon l'invention peut être de type silylé de stéréochimie « *cis »* et « *trans* » et répond à la formule 4 : 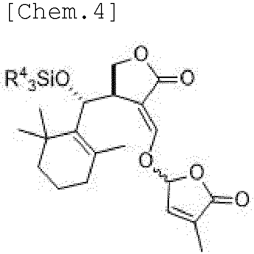 dans laquelle :
- R¹ désigne le groupe OSiR⁴₃,
- R² désigne le groupement méthyle,
- R³ désigne l'atome d'hydrogène H, et
- le cycle carboné à 6 chaînons est de type cyclohexène.

Selon un quatrième mode de réalisation de l'invention, l'analogue de la cannalactone selon l'invention peut être de type alcool de stéréochimie « *cis* » et « *trans* » et répond à la formule 5 : 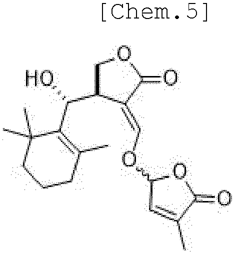 dans laquelle :
- R¹ désigne le groupe hydroxyle OH,
- R² désigne le groupement méthyle,
- R³ désigne l'atome d'hydrogène H, et
- le cycle carboné à 6 chaînons est de type cyclohexène.

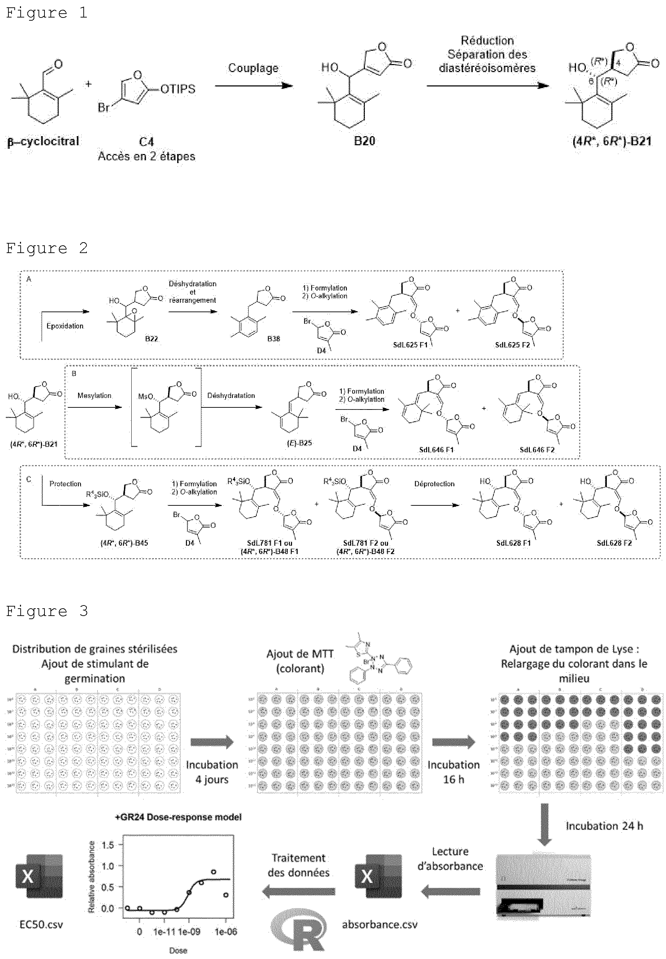

La présente invention a également pour objet un procédé de synthèse d'un analogue
de la cannalactone selon le premier mode de réalisation, caractérisé en ce qu'il comprend les étapes suivantes :
- une réaction A) de couplage du β-cyclocitral commercial avec un bromofurane C4 de formule 6 : 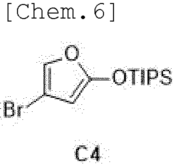 pour obtenir un alcool B20 de formule 7 : 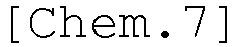 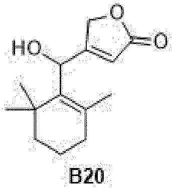
- une étape B) de réduction de l'alcool B20 de formule 7, pour obtenir un mélange de diastéréoisomères de l'alcool allylique, suivie une étape de séparation desdits diastéréoisomères pour retenir le diastéréoisomère (4R*, 6R*)-B21 de formule 8 : 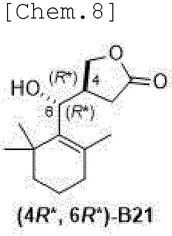 Cette synthèse est illustrée par la Figure 1.
- une étape C2) d'époxydation du diastéréoisomère (4R*, 6R*)-B21 de formule 8 pour obtenir un époxyalcool B22 de formule 9 : 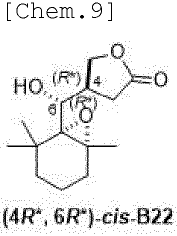
- une étape D2) de déshydratation et réarrangement de l'époxyalcool B22 de formule 9 pour obtenir un composé benzylique B38 de formule 10 : 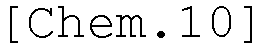 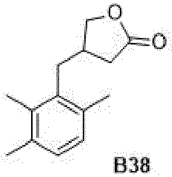
- une étape E2) de formylation en milieu basique avec un formiate d'alkyle du composé benzylique B38 de formule 10 pour obtenir un énol B40 de formule 11 : 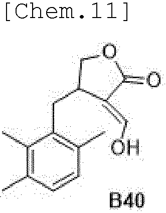
- un étape F2) d'*O*-alkylation de l'énol B40 pour obtenir l'analogue de formule 2 (de type aromatique).

Le formiate d'alkyle (notamment un formiate d'éthyle ou de méthyle) répond à la formule 12

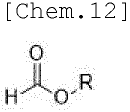

Cette synthèse est illustrée par la [Fig.2] (partie A).

La présente invention a également pour objet un procédé de synthèse d'un analogue
de la cannalactone selon le deuxième mode de réalisation, caractérisé en ce qu'il comprend les étapes suivantes :
- les étapes A et B telles que définies dans le procédé de synthèse d'un analogue
   de la cannalactone selon le premier mode de réalisation, suivies par
- une étape C3) de mésylation du diastéréoisomère (4R*, 6R*)-B21 de formule 8 pour obtenir après déshydratation et réarrangement le diène (E)-B25 de formule 13 : 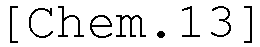 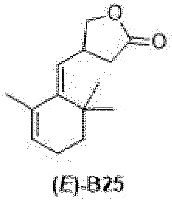
- une étape E3) de formylation en milieu basique du diène (E)-B25 de formule 13 pour obtenir un énol B42 de formule 14 : 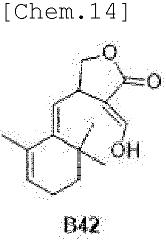 puis
- un étape F3) d'*O*-alkylation de l'énol B42 pour obtenir l'analogue de formule 3 (de type diène).

Cette synthèse est illustrée par la [Fig.2] (partie B).

La présente invention a également pour objet un procédé de synthèse d'un analogue
de la cannalactone selon le troisième mode de réalisation, caractérisé en ce qu'il comprend les étapes suivantes :
- les étapes A et B telles que définies les étapes A et B telles que définies dans le procédé de synthèse d'un analogue de la cannalactone selon le premier mode de réalisation, suivies de
- une étape C4) de protection du diastéréoisomère (4R*, 6R*)-B21 de formule 8 pour obtenir le composé protégé (4R*, 6R*)-B45 de formule 15 : 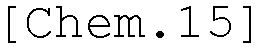 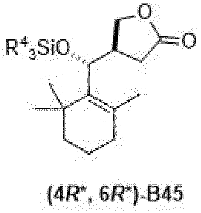
- une étape E4) de formylation en milieu basique du composé protégé de formule 14, pour obtenir un énol (4R*, 6R*)-B46 de formule 16 : 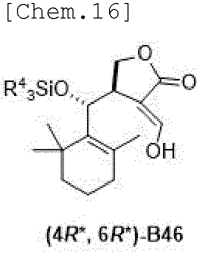
- un étape F4) d'*O*-alkylation de l'énol B46 pour obtenir l'analogue de formule 4 (de type silyle).

Cette synthèse est illustrée par la [Fig.2] (partie C).

La présente invention a également pour objet un procédé de synthèse d'un analogue
de la cannalactone selon le quatrième mode de réalisation, caractérisé en ce qu'il comprend les étapes suivantes :
- les étapes A et B telles que définies les étapes A et B telles que définies dans le procédé de synthèse d'un analogue de la cannalactone selon le premier mode de réalisation, suivies de
- la formation d'un analogue de la cannalactone selon le troisième mode de réalisation, suivie par
- une étape G5) de déprotection et de séparation des diastéréoisomères de l'analogue de formule 4, pour obtenir l'analogue de formule 5 (de type alcool).

Cette synthèse est illustrée également par la Figure 2 (partie C).

La présente invention a encore pour objet l'utilisation d'un analogue de la cannalactone selon l'invention ou telle qu'obtenue selon l'un des procédés de synthèse selon l'invention, en tant que stimulant de germination de graines de plantes parasites.

En particulier, on pourra l'utiliser en tant que stimulant de germination des graines de *P. ramosa* 1 et *P. ramosa* 2a*,* ou pour la germination suicide de plantes parasites de type *Striga, Orobanche* et *Phelipanche.*

### Brève description des figures

D'autres caractéristiques et avantages de l'invention pourront encore apparaître à l'homme du métier à la lecture des exemples ci-dessous, donnés à titre illustratif et non limitatif et illustrés par les figures annexées :
La Figure 1 représente le schéma de la synthèse du diastéréoisomère (4 *R**, 6 *R**)-B21 de formule (8) réalisée à l'exemple 1 ;
La Figure 2 représente un schéma global des synthèses analogues de la cannalactone réalisées dans les exemples 2 à 5, à partir du diastéréoisomère (4 *R*,* 6*R** )-B21 de formule (8) obtenu à l'exemple 1 ;
La Figure 3 est une représentation schématique du protocole des tests de germination mis en œuvre à l'exemple 6.
La Figure 4 est une courbe de dose-réponse du (±)-GR24 et de la (+)cannalactone sur la stimulation de germination des graines de *P. ramosa* 2a et 1.
La Figure 5 est une courbe histogramme montrant les activités maximales de stimulation de la germination des analogues de la cannalactone selon l'invention des exemples 3 à 6 (comportant le cycle A) comparées à celles de la (+)-cannalactone et du (±)-GR24 sur les graines de *P*. *ramosa* 1 et 2a. Les données sont des moyennes ± SE (n = 6-12 répétitions).
La Figure 6 est une courbe histogramme montrant les concentrations efficaces médianes EC₅₀ (en mol.L⁻¹) des analogues de la cannalactone selon l'invention des exemples 3 à 6, comparées à celles de la (+)-cannalactone et du (±)-GR24 sur la stimulation de germination des graines de *P*. *ramosa* 1 et 2a. Les données sont des moyennes ± SE (n = 6-12 répétitions).
La Figure 7 est une courbe histogramme montrant l'évolution du rapport de la concentration efficace médiane EC₅₀ des analogues de la cannalactone selon l'invention des exemples 3 à 6 (comportant le cycle A) comparé à celle de la (+)cannalactone et du (±)-GR24, sur la stimulation de germination des graines de *P.ramosa* 1 et 2a.

r_{EC50} = EC₅₀(*P. ramosa* 1)/EC₅₀(*P. ramosa* 2a) pour chaque analogue.

### EXEMPLES

### Solvants et réactifs

Les réactifs chimiques, sont des produits commerciaux notamment commercialisés par les sociétés Sigma Aldrich, Alfa Aesar, Acros Organics et TCI. Ils ont été utilisés sans purification supplémentaire.

Les solvants anhydres de qualité analytique sont des produits commerciaux notamment commercialisés par les sociétés Sigma Aldrich et Acros Organics.
Le tétrahydrofurane (THF) a été distillé sous argon sur sodium en présence de benzophénone. Les solvants deutérés sont commercialisés par la société Eurisotop.

### Matériels et Méthodes

Les réactions non aqueuses ont été effectuées sous atmosphère inerte (argon ou azote), en utilisant les techniques standards de manipulation des composés sensibles à l'air et à l'humidité.

Toutes les réactions ont été suivies par chromatographie sur couche mince (CCM) sur des plaques d'aluminium pré-enduites de gel de silice (commercialisé par la société Merck sous la dénomination commerciale 60 F254 avec détection par UV à courte longueur d'onde (c'est-à-dire λ = 254 nm), et/ou par coloration avec une solution de KMnO₄ [1 % (p/p)] dans de l'eau ou une solution de vanilline [1 % (p/p)] dans une solution éthanoïque à 1 % (v/v) d'acide phosphorique.

La plupart des séparations ont été effectuées dans des conditions de Flashchromatographie sur gel de silice à l'aide d'une cartouche garnie (gel de silice de 40-63 µm) à pression moyenne (20 psi) avec pompe et collecteur de fraction Armen ou un appareil Buchi Pure C-805 Flash.

Certaines séparations ont été réalisées sur chromatographie préparative en couche mince (PTLC) (gel de silice Merck 60 F254 sur verre).

Les spectres RMN ¹H ont été enregistrées sur des spectromètres Bruker à 300, 500 ou 700 MHz. Les spectres RMN ¹³C ont été enregistrées sur les mêmes instruments à 75, 125 ou 175 MHz. Les déplacements chimiques δ sont exprimés en parties par million (ppm) avec les signaux des solvants résiduels comme référence interne (δ = 7,24 pour la RMN ¹H et 77,23 pour la RMN ¹³C dans CDCl₃). Pour la RMN ¹H, les spectres sont décrits comme suit : déplacement chimique, intégration, multiplicité (s = singulet, d = doublet, t = triplet, q = quadruplet, quint = quintuplet, sext = sextuplet, dd = doublet de doublet, dt = doublet de triplet, m = multiplet), constante de couplage en Herz (J) et attribution. Toutes les attributions RMN sont basées sur des expériences RMN 2D COSY, HSQC et HMBC. Des expériences NOESY ont été enregistrées pour confirmer les configurations des doubles liaisons.

Les spectres IR ont été enregistrés sur un spectromètre PerkinElmer Spectrum 100 FT-IR, les absorptions étant données en centimètres⁻¹ (cm⁻¹).

Les spectres de masse à basse résolution ont été déterminés par ionisation par électronébulisation sur un système UPLC Acquity de Waters, combiné à un détecteur à photodiodes (PDA), un détecteur de diffusion de la lumière par évaporation (ELSD) et par un spectromètre de masse avec un détecteur quadripolaire en tandem (TQD). Les tampons et les phases mobiles aqueuses pour l'UPLC ont été préparés en utilisant de l'eau purifiée avec un système Milli-Q.

Les spectres de masse à haute résolution ont été obtenus avec le dispositif UPLC Waters Acquity (par injection directe ou avec une colonne BEH C₁₈ 2,1 Å ~50 mm, 1,7 µm) combiné à un PDA et un instrument de masse Waters LCT Premier XE [ESI avec un analyseur de temps de vol (ToF)].

### EXEMPLE 1 : synthèse du diastéréoisomère (4 R*, 6 R* )-B21 de formule (8)(voie d'accès illustrée par la Figure 1)

### 4-Bromofuran-2(5H)-one

A une solution de furan-2,4(3*H*,5*H*)-dione (1,0 g, 10,00 mmol) dans du CH₂Cl₂ (22 mL) et du DMF (1 mL) à 0 °C a été ajouté du dibromure d'oxalyle (2,6 g, 12,00 mmol, 1,2 équiv.). Le mélange a été agité pendant 1 h à 0 °C et réchauffé progressivement à température ambiante pendant 2 h. Le mélange réactionnel a été dilué avec de l'eau (50 mL) et extrait avec de l'EtOAc (3 x 20 mL). Les phases organiques combinées ont été lavées avec de l'eau (2 x 30 mL), une solution aqueuse de NaHCO₃ saturée (2 x 30 mL) et de la saumure (2 x 30 mL) et séchées sur Na₂SO₄. Les solvants ont été éliminés pour obtenir le produit brut 4-Bromofuran-2(5*H*)-one (1,61 g, quantitatif) sous la forme d'un solide brun. Les analyses chimiques sont en accord avec la littérature [15].

### (4-Bromofuran-2-yl)oxytriisopropylsilane (C4)

A une solution de 4-bromofuran-2(5*H*)-one (720,4 mg, 4,40 mmol) dans du CH₂ Cl₂ (6,2 mL) sous argon à 0 °C, a été ajouté de la Et₃N (626,4 mg, 6,20 mmol, 1,4 équiv.). Le mélange a été agité pendant 1 minute, puis du trifluorométhanesulfonate de triisopropylsilyle (TIPSOTf) (1,42 g, 4,60 mmol, 1,05 équiv.) a été ajouté goutte à goutte à 0 °C. Le mélange ainsi obtenu a été agité pendant 10 minutes à 0 °C, puis réchauffé à la température ambiante et agité pendant encore 1 h 30. Le mélange a été dilué avec de l'heptane (10 mL), lavé avec une solution aqueuse de NaHCO₃ saturée (2 x 10 mL), de l'eau (2 x 10 mL) et de la saumure (2 x 10 mL). La phase organique a été séchée sur Na₂SO₄. Les solvants ont été éliminés pour obtenir le bromofurane C4 (1,4 g, quantitatif) sous forme d'huile brune. Les analyses chimiques sont en accord avec la littérature [15].

### 4-[Hydroxy(8,12,12-triméthylcyclohex-7-en-6-yl)méthyl]furan-2(5 H )-one (B20)

A une solution de C4 (89,9 mg, 0,28 mmol) dans du THF anhydre (1,8 mL) sous argon à -78 °C, une solution de n-BuLi a été ajoutée goutte à goutte (0,3 mL, 0,30 mmol, 0,98 M, 1,1 équiv.). Le mélange ainsi obtenu a été agité à -78 °C pendant 30 minutes. Un mélange de β-cyclocitral (51,6 mg, 0,34 mmol, 1,2 équiv.) dans du THF anhydre (2 mL) y a été ensuite ajouté. Le mélange réactionnel a été agité pendant 2 h à -78 °C et 12 h à température ambiante. Le mélange a été hydrolysé avec une solution aqueuse de NH₄Cl saturée (5 mL) et une solution aqueuse de HCl (5 mL, 2 M). La phase organique a été séparée et la phase aqueuse a été extraite avec EtOAc (3 x 5 mL). Les phases organiques combinées ont été lavées avec de l'eau (2 x 5 mL), une solution aqueuse de NaHCO₃ saturée (2 x 5 mL), de l'eau (2 x 5 mL) et de la saumure (2 x 5 mL), puis séchées sur Na₂SO₄. Les solvants ont été éliminés et le produit brut a été purifié par chromatographie sur gel de silice (heptane/EtOAc, 95:5 à 60:40 pendant 20 min) pour obtenir le produit pur B20 (24,5 mg, 37%) sous forme d'huile brune :
B20

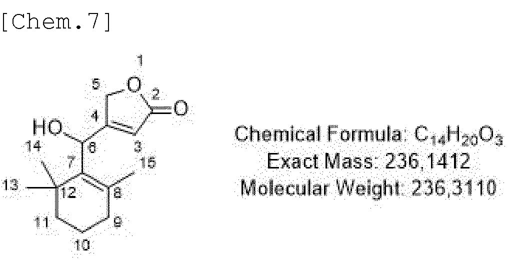

RMN ¹H (500 MHz, CDCl₃) δ 5.91 (1H, d, J = 1.5 Hz, H-3), 5.10 (1H, s, H-6), 4.88 (1H, d, J = 18.0 Hz, H-5a), 4.71 (1H, d, J = 18.0 Hz, H-5b), 1.96 (2H, t, J = 6.0 Hz, H-9), 1.61 (3H, s, H-15), 1.59-1.55 (2H, m, H-10), 1.50-1.46 (2H, m, H-11), 1.13 (3H, s, H-13 ou H-14), 0.98 (3H, s, H-13 ou H-14).

RMN ¹³C (75 MHz, CDCl₃) δ 174.1 (C-2), 174.0 (C-4), 138.7 (C-7), 136.6 (C-8), 115.0 (C-3), 71.9 (C-5), 67.8 (C-6), 39.5 (C-11), 35.0 (C-12), 33.7 (C-9), 28.9 (C-13 ou C-14), 28.5 (C-13 ou C-14), 21.4 (C-15), 19.3 (C-10).

IR (film) νₘₐₓ 3471, 2932, 1777, 1741, 1637, 1447, 1268, 1111, 1028 cm⁻¹.

HRESIMS *m*/*z* 237.1491 [M + H]⁺ (calc. pour C₁₄H₂₁O₃, 237.1491).

### 4-[Hydroxy(8,12,12-triméthylcyclohex-7-en-6-yl)méthyl]dihydrofuran-2(3 H )one (B21)

A une solution de B20 (696.4 mg, 2.95 mmol) dans du méthanol (45 mL) à 15 °C, a été ajouté du NiCl₂ (350.9 mg, 1.48 mmol, 0.5 équiv.) puis du borohydrure de sodium (358,3 mg, 9,47 mmol, 3,2 équiv.) par portions. Le mélange a été agité à 15 °C jusqu'à ce que l'analyse TLC indique une conversion complète. Le mélange réactionnel a été hydrolysé avec une solution aqueuse de HCl (50 mL, 2 M). La phase aqueuse a été extraite avec CH₂Cl₂ (3 x 20 mL). Les phases organiques combinées ont été séchées sur Na₂SO₄ et les solvants ont été éliminés. Le mélange obtenu a ensuite été purifié par chromatographie sur gel de silice (CH₂Cl₂/EtOAc, 100:0 à 90:10 pendant 30 min) pour obtenir le produit pur (4*R**, 6*R**)-B21 de formule 8 (327,4 mg, 47%) sous forme d'huile jaune et (4*R**, 6*S**)-B21 de formule 17 (131,9 mg, 19%) sous forme de solide blanc.

### (4R*, 6R*)-B21

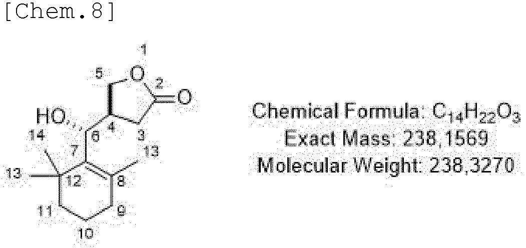

RMN ¹H (500 MHz, CDCl₃) δ 4.50 (1H, dd, J = 9.5, 7.0 Hz, H-5a), 4.28 (1H, dd, J =
9.5, 7.0 Hz, H-5b), 4.21 (1H, d, J = 9.5 Hz, H-6), 3.21 (1H, sext, J = 9.5 Hz, H-4), 2.41
(1H, dd, J = 17.5, 8.5 Hz, H-3a), 2.18 (1H, dd, J = 17.5, 8.5 Hz, H-3b), 1.96 (2H, q, J = 5.5 Hz, H-9), 1.81 (3H, s, H-15), 1.59-1.52 (2H, m, H-10), 1.48-1.45 (1H, m, H-11a), 1.40-1.35 (1H, m, H-11b), 1.08 (3H, s, H-13 ou H-14), 0.98 (3H, s, H-13 ou H-14).

RMN ¹³C (125 MHz, CDCl₃) δ 177.1 (C-2), 138.4 (C-7), 135.1 (C-8), 72.9 (C-5), 72.6 (C-6), 41.3 (C-4), 40.4(C-11), 35.0 (C-12), 34.6 (C-9), 32.4 (C-3), 29.2 (C-13 ou C-14), 29.1 (C-13 ou C-14), 21.3 (C-15), 19.4 (C-10).

IR (film) νₘₐₓ 3464, 2928, 1768, 1551, 1365, 1263, 1178, 1048, 1001, 892 cm⁻¹.

HRESIMS *m*/*z* 239.1640 [M + H]⁺ (calc. pour C₁₄H₂₃O₃, 239.1647).

### (4R*, 6S*)-B21

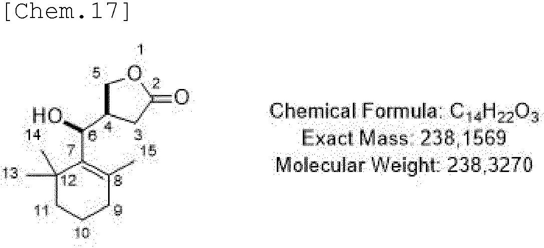

RMN ¹H (500 MHz, CDCl₃) δ 4.17 (1H, dd, J = 9.0, 7.0 Hz, H-5a), 4.16 (1H, d, J
= 9.0 Hz, H-6), 3.94 (1H, dd, J = 9.0, 7.0 Hz, H-5b), 3.19 (1H, sext, J = 9.0 Hz, H-4), 2.72 (1H, dd, J = 17.5, 7.5 Hz, H-3a), 2.57 (1H, dd, J = 17.5, 7.5 Hz, H-3b), 1.96 (2H, q, J = 5.0 Hz, H-9), 1.80 (3H, s, H-15), 1.59-1.51 (2H, m, H-10), 1.47-1.44 (1H, m, H-11a), 1.40-1.34 (1H, m, H-11b), 1.08 (3H, s, H-13 ou H-14), 0.97 (3H, s, H-13 ou H-14).

RMN ¹³C (125 MHz, CDCl₃) δ 177.5 (C-2), 138.2 (C-7), 135.1 (C-8), 72.3 (C-6), 70.5 (C-5), 41.5 (C-4), 40.4 (C-11), 35.1 (C-12), 34.6 (C-9), 33.8 (C-3), 29.2 (C-13 ou C-14), 28.8 (C-13 ou C-14), 21.4 (C-15), 19.4 (C-10).

IR (film) νₘₐₓ 3481, 2925, 2870, 1774, 1547, 1465, 1373, 1258, 1176, 1092, 1033, 1011, 890, 795 cm⁻¹.

HRESIMS *m*/*z* 239.1638 [M + H]⁺ (calc. pour C₁₄H₂₃O₃ 239.1647).

### EXEMPLE 2 : synthèse d'analogues de la cannalactone de type aromatique selon l'invention, à partir du diastéréoisomère (4 R*, 6 R* )-B21 de l'exemple 1 (de formule 8)

### (4 R *)-[(6 R *)-hydroxy(8,12,12-triméthyl-7-oxabicyclo[4.1.0]heptan-6yl)méthyl]dihydrofuran-2(3 H )-one ((4 R *, 6 R *)- cis -B22)

Cette synthèse est illustrée par la Figure 2.

A une solution de (4R*, 6R*)-B21 (100.9 mg, 0.420 mmol) dans du toluène anhydre (5.1 mL), a été ajouté une solution de VO(acac)₂ (3.9 mg, 0.015 mmol, 0.04 équiv.) dans du toluène anhydre (0.2 mL). Du *tert*-Butyl hydroperoxide (TBHP) (0,11 mL, 5,5 M, 0,590 mmol, 1,4 équiv.) Le mélange obtenu a été agité à température ambiante pendant 1 h. Le mélange réactionnel a été hydrolysé avec une solution aqueuse de NaOH (5 mL, 5%). La phase aqueuse a été extraite avec de l'heptane et de l'EtOAc (2:1) (3 x 10 mL). Les phases organiques combinées ont été lavées avec de la saumure (2 x 10 mL), séchées sur Na₂SO₄ et les solvants ont été éliminés pour obtenir le produit pur (4*R**, 6*R**)-*cis-*B22 de formule 9 (116,4 mg, quantitatif) sous forme d'huile incolore utilisée dans l'étape suivante sans purification.

### (4R*, 6R*)-cis-B22

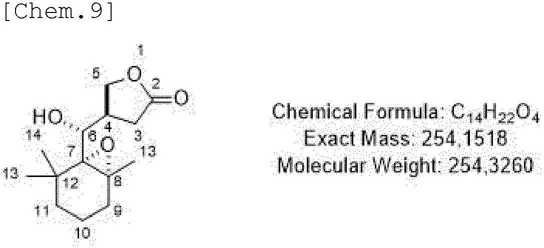

RMN ¹H (300 MHz, CDCl₃) δ 4.40 (1H, dd, J = 9.5, 8.0 Hz, H-5a), 4.31 (1H, dd, J
= 9.5, 8.0 Hz, H-5b), 3.96 (1H, d, J = 8.0 Hz, H-6), 2.94 (1H, sext, J = 8.0 Hz, H-4),
2.59 (1H, dd, J = 17.0, 9.0 Hz, H-3a), 2.48 (1H, dd, J = 17.0, 9.0 Hz, H-3b), 1.90-1.80
(1H, m, H-9a), 1.78-1.69 (1H, m, H-9b), 1.39 (3H, s, H-15), 1.36-1.32 (2H, m, H-10), 1.25-1.22 (1H, m, H-11a), 1.06 (3H, s, H-13 ou H-14), 1.05-1.03 (1H, m, H-11b), 1.02 (3H, s, H-13 ou H-14).

RMN ¹³C (75 MHz, CDCl₃) δ 176.4 (C-2), 71.1 (C-5), 70.5 (C-6), 70.4 (C-7), 66.3 (C-8), 40.0 (C-4), 37.6 (C-11), 33.9 (C-13), 33.3 (C-3), 31.8 (C-9), 25.6 (C-13 et C-14), 22.2 (C-15), 17.0 (C-10).

IR (film) νₘₐₓ 3464, 2928, 1768, 1551, 1365, 1263, 1178, 1048, 1001, 892 cm⁻¹.

HRESIMS *m*/*z* 255.1607 [M + H]⁺ (calc. pour C₁₄H₂₃O₄, 255.1596)

### 4-(8,11,12-Triméthylbenzyl)dihydrofuran-2(3 H )-one (B38)

A une solution de B22 (62,8 mg, 0,21 mmol) dans du toluène (15 mL) à 120 °C, a été ajouté de l'acide para-toluène sulfonique (APTS) (4,8 mg, 0,02 mmol, 10 mol%) et la réaction a été agitée pendant 2 h à cette température avant d'être refroidie à température ambiante. Le mélange a été dilué avec de l'eau (15 ml), extrait avec CH₂Cl₂ (3 x 10 ml) et séché sur Na₂SO₄. Les solvants ont été éliminés pour obtenir le produit brut B38 (62,1 mg, quantitatif) de formule 10. Le produit brut a été utilisé sans purification dans l'étape suivante.

### B38

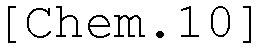

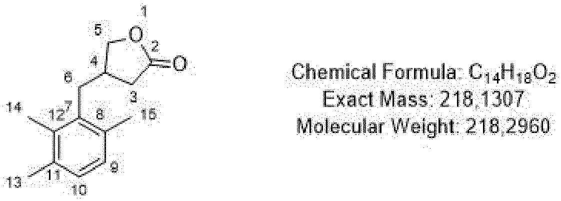

RMN ¹H (500 MHz, CDCl₃) δ 6.93 (2H, q, J = 8.0 Hz, H-9 et H-10), 4.26 (1H, dd,
J = 9.0, 5.5 Hz, H-5a),,4.02 (1H, dd, J = 9.0, 5.5 Hz, H-5b), 2.86-2.84 (2H, m, H-6),
2.78 (1H, sext, J = 8.0 Hz, H-4), 2.57 (1H, dd, J = 17.0, 8.0 Hz, H-3a), 2.28 (1H, dd, J = 17.0, 8.0 Hz, H-3b), 2.27 (3H, s, H-14), 2.23 (3H, s, H-13 ou H-15), 2.20 (3H, s, H-13 ou H-15).

RMN ¹³C (125 MHz, CDCl₃) δ 177.2 (C-2), 135.3 (C-11 or C-8), 135.2 (C-11 or
C-8), 135.0 (C-7), 134.1, (C-12), 128.5 (C-9 or C-10), 128.2 (C-9 or C-10), 72.7 (C-5), 36.2 (C-4), 34.7 (C-3), 32.2 (C-6), 21.0 (C-13 ou C-15), 20.8 (C-13 ou C-15), 16.3 (C-14).

IR (film) νₘₐₓ 2932, 1777, 1734, 1464, 1379, 1169, 1014, 810 cm⁻¹.

HRESIMS m/z 219.1377 [M + H]⁺ (calc. pour C₁₄H₁₉O₂, 219.1385).

### ( E )-3-(Hydroxyméthylène)-4-(8,11,12-triméthylbenzyl)dihydrofuran-2(3 H )one (B40)

A une solution de B38 (62.1 mg, 0.21 mmol) dans du THF anhydre (2.1 mL) à 0 °C sous argon, a été ajouté du formiate d'éthyle (0.16 mL, 2.10 mmol, 10.0 équiv.) et du *tert*-BuOK (235.5 mg, 2.10 mmol, 10.0 équiv.). Le mélange a été agité pendant 30 minutes à 0 °C, puis a été laissé se réchauffer à température ambiante et on l'a agité pendant 1 h. Le mélange réactionnel a été hydrolysé avec une solution aqueuse de HCl (3 mL, 1 M). Le mélange a été extrait avec de l'EtOAc (3 x 5 mL), lavé avec de la saumure (2 x 5 mL), séché sur Na₂SO₄ et les solvants ont été éliminés. Le produit brut a été purifié par chromatographie sur gel de silice (heptane/EtOAc, 70:30) pour obtenir le produit pur B40 de formule 11 (31,4 mg, 61% en 2 étapes).

### B40

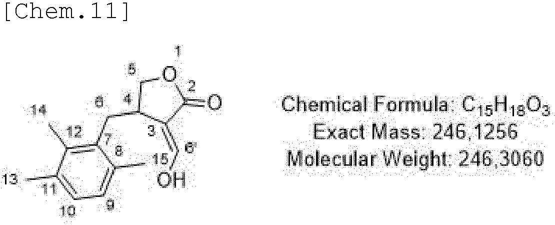

IR (film) νmax 3673, 2969, 2922, 1778, 1745, 1462, 1385, 1262, 1169, 1051, 799 cm⁻¹.

HRESIMS *m*/*z* 245.1176 [M - H]⁺ (calc. pour C15H17O3, 245.1178).

### (±)-SdL625

A une solution de B40 (30,0 mg, 0,12 mmol) dans de l'acétone anhydre (1,2 mL) sous argon, a été ajouté du K₂CO₃ anhydre (34,5 mg, 0,24 mmol, 2,0 équiv.). A ce mélange a été ajouté le 5-bromo-3-méthylfurane-2(5*H*)-one D4 [16] (32,3 mg, 0,18 mmol, 1,5 équiv.) dans de l'acétone anhydre (1,2 ml). La réaction a été agitée pendant 2 h à température ambiante. Les solvants ont été éliminés et le mélange a été dissous dans de l'EtOAc (5 mL) et filtré pour éliminer les sels. Les solvants ont été éliminés et le produit brut a été purifié par PTLC (heptane/EtOAc, 50:50) pour obtenir le produit pur (±)-SdL625 F1 de formule 18 (8,3 mg, 17%) et (±)-SdL625 F2 de formule 19 (7,1 mg, 20%) sous forme d'huiles incolores. (±)-SdL625 F1 et (±)-SdL625 F2 sont des analogues de la cannalactone de type aromatique répondant à la formule générale 2.

### (±)-SdL625 F1

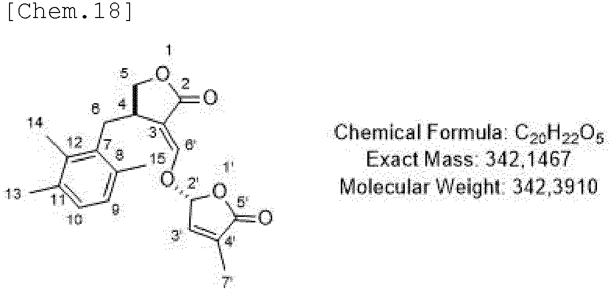

RMN ¹H (700 MHz, CDCl3) δ 7,41 (1H, d, J = 1.5 Hz, H-6'), 6.94 (1H, d, J = 8.0
Hz, H-9 or H-10), 6.90 (1H, d, J = 8.0 Hz, H-9 or H-10), 6.57 (1H, t, J = 1.5 Hz, H-3'),
5.81 (1H, t, J = 1.5 Hz, H-2'), 4.16 (1H, dd, J = 9.0, 7.0 Hz, H-5a), 4.07 (1H, dd, J =
9.0, 1.5 Hz, H-5b), 3.50 (1H, q, J = 7.5 Hz, H-4), 3.01 (1H, dd, J = 14.0, 8.5 Hz, H-6a), 2.88 (1H, dd, J = 14.0, 8.5 Hz, H-6a), 2.26 (3H, s, H-13 ou H-15), 2.23 (3H, s, H-13 ou H-15), 2.20 (3H, s, H-14), 1.97 (3H, s, H-7').

RMN ¹³C (175 MHz, CDCl3) δ 171.9 (C-2), 170.4 (C-5'), 150.9 (C-6'), 141.1 (C-3'), 135.6 (C-4'), 135.6 (C-12), 135.3 (C-7), 134.8 (C-8 ou C-11), 134.7 (C-8 ou C-11), 128.3 (C-9 ou C-10), 127.9 (C-9 ou C-10), 112.2 (C-3), 100.3 (C-2'), 71.0 (C-5), 37.1 (C-4), 33.0 (C-6), 21.0 (C-13 ou C-15), 21.0 (C-13 ou C-15), 16.4 (C-14), 10.9 (C-7').

IR (film) νmax 2969, 2924, 2860, 1785, 1754, 1681, 1465, 1340, 1257, 1174, 1084, 1021, 953, 868, 794 cm⁻¹.

HRESIMS *m*/*z* 343.1538 [M + H]⁺ (calc. pour C20H23O5, 343.1545).

### (±)-SdL625 F2

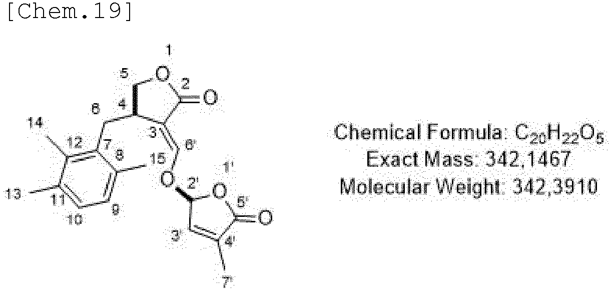

RMN ¹H (700 MHz, CDCl3) δ 7,38 (1H, s, H-6'), 6.88 (1H, d, J = 8.0 Hz, H-9 ou
H-10), 6.84 (1H, d, J = 8.0 Hz, H-9 ou H-10), 6.68 (1H, s, H-3'), 5.93 (1H, s, H-2'), 4.15 (1H, dd, J = 9.5, 7.0 Hz, H-5a), 4.07 (1H, dd, J = 9.5, 2.0 Hz, H-5b), 3.50 (1H, q, J = 8.0 Hz, H-4), 3.02 (1H, dd, J = 14.0, 7.5 Hz, H-6a), 2.86 (1H, dd, J = 14.0, 9.5 Hz, H-6a), 2.24 (3H, s, H-13 ou H-15), 2.20 (3H, s, H-13 ou H-15), 2.19 (3H, s, H-14), 1.99 (3H, s, H-7').

RMN ¹³C (175 MHz, CDCl3) δ 171.9 (C-2), 170.2 (C-5'), 150.3 (C-6'), 140.7 (C-3'),
136.1 (C-4'), 135.4 (C-12), 135.1 (C-7), 134.8 (C-8 ou C-11), 134.5 (C-8 ou C-11), 128.3 (C-9 ou C-10), 127.9 (C-9 ou C-10), 112.1 (C-3), 100.0 (C-2'), 71.0 (C-5), 37.1 (C-4), 32.9 (C-6), 21.0 (C-13 ou C-15), 21.0 (C-13 ou C-15), 16.3 (C-14), 11.0 (C-7').

IR (film) νmax 2966, 2922, 2848, 1781, 1756, 1682, 1347, 1260, 1184, 1090, 1024, 950, 797 cm⁻¹.

HRESIMS *m*/*z* 343.1540 [M + H]⁺ (calc. pour C20H23O5, 343.1545).

### EXEMPLE 3 : synthèse d'analogues de la cannalactone de type diène selon l'invention, à partir du diastéréoisomère (4 R*, 6 R* )-B21 de l'exemple 1

Cette synthèse est illustrée par la Figure 2.

### ( E )-4-[(8,12,12-Triméthylcyclohex-8-en-6-ylidène)méthyl]dihydrofuran-2(3 H )-one (( E )-B25)

A une solution de B21 (200,0 mg, 0,84 mmol) dans la pyridine (6,8 mL), a été ajouté du DMAP (4-diméthylaminopyridine 5,1 mg, 0,04 mol, 5 mol%) et du MsCl (0,3 mL, 3,40 mmol, 4,0 equiv.). Le mélange a été agité pendant une nuit à température
ambiante. Le mélange réactionnel a été co-évaporé avec du toluène. Le mélange a été dilué avec CH₂Cl₂ (10 mL), lavé avec de l'eau (2 x 5 mL) et de la saumure (2 x 5 mL) et séché avec Na₂SO₄. Les solvants ont été éliminés et le produit brut a été purifié par chromatographie sur gel de silice (heptane/EtOAc, 80:20) pour obtenir le produit pur (*E*)-B25 de formule 13 (142,2 mg, 77%).

### (E)-B25

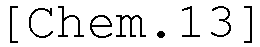

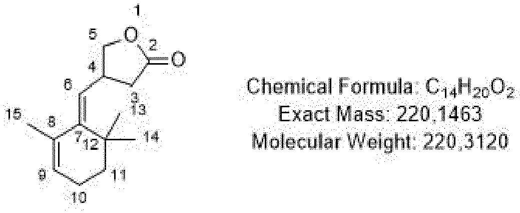

RMN ¹H (500 MHz, CDCl3) δ 5.73 (1H, t, J = 4.5 Hz, H-9), 5.18 (1H, d, J = 10.0
Hz, H-6), 4.44 (1H, t, J = 8.0 Hz, H-5a), 3.93 (1H, t, J = 8.0 Hz, H-5b), 3.69 (1H, m,
H-4), 2.69 (1H, dd, J = 17.5, 8.0 Hz, H-3a), 2.30 (1H, dd, J = 17.0, 9.5 Hz, H-3b), 2.05
(1H, m, 2 H-11), 1.78 (3H, s, H-15), 1.46 (2H, t, J = 5.6 Hz, H-10), 1.20 (6H, s, H-13
et H-14).

RMN ¹³C (125 MHz, CDCl3) δ 176.9 (C-2), 147.4 (C-7), 132.8 (C-8), 128.5 (C-9),
122.8 (C-6), 73.8 (C-5), 40.3 (C-11), 36.7 (C-4), 36.5 (C-3), 35.0 (C-12), 29.5 (C-13
ou C-14), 29.1 (C-13 ou C-14), 22.9 (C-10), 22.0 (C-15).

IR (film) νmax 2932, 2857, 1779, 1545, 1469, 1380, 1265, 1178, 1042, 1001, 882,
739 cm⁻¹.

HRESIMS m/z 221.1542 [M + H]⁺ (calc. pour C14H21O2, 221.1542).

### ( E )-3-(Hydroxyméthylène)-4-[( E )-(8,12,12-triméthylcyclohex-8-èn-6-ylidene)méthyl]dihydrofuran-2(3 H )-one (B42)

A une solution de (E)-B25 (19,0 mg, 0,09 mmol) dans du THF anhydre (0,9 mL) à 0 °C sous argon, est ajouté du formiate d'éthyle (70 µL, 0,90 mmol, 10,0 équiv.) et du tert-BuOK (101,0 mg, 0,90 mmol, 10,0 équiv.). Le mélange a été agité pendant 30 minutes à 0 °C, puis a été laissé se réchauffer à température ambiante et a été agité pendant 1 h. Le mélange réactionnel a été hydrolysé avec une solution aqueuse de HCl mL, 1 M). Le mélange a été extrait avec de l'EtOAc (5 mL), lavé avec de la saumure x 5 mL) et séché sur Na₂SO₄. Les solvants ont été éliminés et le produit brut a été purifié par chromatographie sur gel de silice (heptane/EtOAc, 70:30) pour obtenir le produit pur B42 de formule 14 (14,8 mg, 66%) sous forme d'huile incolore.

### B42

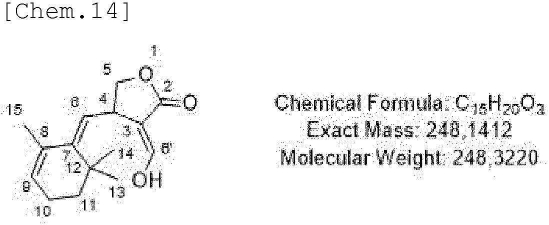

IR (film) νmax 3664, 2975, 2919, 1734, 1396, 1056 cm⁻¹.

HRESIMS *m*/*z* 247.1332 [M + H]⁺ (calc. pour C15H19O3, 247.1334).

### (±) -SdL646

A une solution de B42 (27.7 mg, 0.11 mmol) dans de l'acétone anhydre (1.1 mL) sous argon, a été ajouté du K₂CO₃ anhydre (32.3 mg, 0.22 mmol, 2.0 équiv.). A ce mélange a été additionné du 5-bromo-3-méthylfurane-2(5*H*)-one D4 [16] (30,1 mg, 0,17 mmol, 1,5 équiv.) dans de l'acétone anhydre (1,1 ml). La réaction a été agitée pendant 2 h à température ambiante. Les solvants ont été éliminés et le produit brut a été dissous dans de l'EtOAc (5 mL) et filtré pour éliminer les sels. Les solvants ont été éliminés et le produit brut a été purifié par PTLC (heptane/EtOAc, 50:50) pour obtenir le produit pur (±)-SdL646 F1 de formule 20 (10,9 mg, 19%) et (±)-SdL646 F2 de formule 21 (7,3 mg, 29%) sous forme d'huiles incolores. (±)-SdL646 F1 et (±)SdL646 F2 sont des analogues de la cannalactone de type diène répondant à la formule générale 3.

### (±)-SdL646 F1

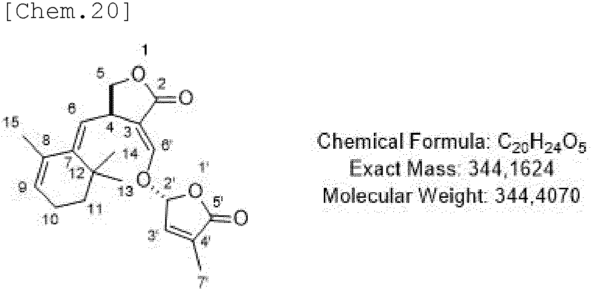

RMN ¹H (700 MHz, CDCl₃) δ 7,48 (1H, d, J = 2.0 Hz, H-6'), 6.80 (1H, t, J = 1.5 Hz,
H-3'), 6.06 (1H, s, H-2'), 5.70 (1H, t, J = 4.5 Hz, H-9), 5.23 (1H, d, J = 10.0 Hz, H-6),
4.51 (1H, q, J = 8.5 Hz, H-5a), 4.49-4.47 (1H, m, H-4), 3.98 (1H, dd, J = 8.5, 4.5 Hz, H-5b), 2.11-2.05 (2H, m, H-10), 1.97 (3H, s, H-7'), 1.75 (3H, s, H-15), 1.54-1.50 (1H,
m, H-11a), 1.39-1.36 (1H, m, H-11b), 1.20 (3H, s, H-13 ou H-14), 1.13 (3H, s, H-13 ou H-14).

RMN ¹³C (175 MHz, CDCl₃) δ 171.8 (C-2), 170.4 (C-5'), 151.2 (C-6'), 145.3 (C-7), 141.0 (C-3'), 135.9 (C-4'), 133.0 (C-8), 127.8 (C-9), 123.4 (C-6), 112.5 (C-3), 100.5
(C-2'), 72.5 (C-5), 40.3 (C-11), 37.4 (C-4), 34.7 (C-12), 30.8 (C-13 ou C-14), 27.2 (C-13 ou C-14), 22.9 (C-10), 22.1 (C-15), 10.9 (C-7').

IR (film) νₘₐₓ 2969, 2925, 2848, 1782, 1757, 1679, 1471, 1344, 1184, 1088, 1029, 1007, 953 cm⁻¹.

HRESIMS *m*/*z* 345.1697 [M + H]⁺ (calc. pour C₂₀H₂₅O₅, 345.1702).

### (±)-SdL646 F2

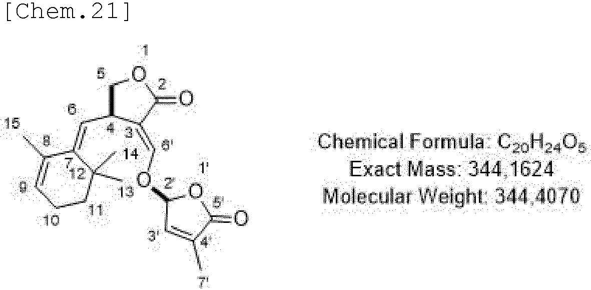

RMN ¹H (700 MHz, CDCl₃) δ 7,45 (1H, d, J = 2.5 Hz, H-6'), 6.81 (1H, t, J = 1.5
Hz, H-3'), 6.07 (1H, t, J = 1.5 Hz, H-2'), 5.67 (1H, t, J = 4.0 Hz, H-9), 5.22 (1H, d, J =
10.0 Hz, H-6), 4.51 (1H, q, J = 8.5 Hz, H-5a), 4.49-4.46 (1H, m, H-4), 3.97 (1H, dd, J
= 8.5, 5.5 Hz, H-5b), 2.09-2.04 (2H, m, H-10), 1.96 (3H, s, H-7'), 1.70 (3H, s, H-15), 1.54-1.50 (1H, m, H-11a), 1.38-1.35 (1H, m, H-11b), 1.21 (3H, s, H-13 ou H-14), 1.15 (3H, s, H-13 ou H-14).

RMN ¹³C (175 MHz, CDCl3) δ 171.8 (C-2), 170.3 (C-5'), 151.0 (C-6'), 145.4 (C-7),
141.0 (C-3'), 136.0 (C-4'), 133.1 (C-8), 127.5 (C-9), 123.2 (C-6), 112.7 (C-3), 100.3 (C-2'), 72.3 (C-5), 40.3 (C-11), 37.6 (C-4), 34.7 (C-12), 30.9 (C-13 ou C-14), 27.1 (C-13 ou C-14), 22.9 (C-10), 21.9 (C-15), 10.9 (C-7').

IR (film) νmax 2963, 2922, 2851, 1782, 1756, 1679, 1453, 1341, 1260, 1184, 1084, 1025, 1009,
953 cm⁻¹.

HRESIMS *m*/*z* 345.1703 [M + H]⁺ (calc. pour C20H25O5, 345.1702).

### EXEMPLE 4 : synthèse d'analogues de la cannalactone de type silylé selon l'invention, à partir du diastéréoisomère (4 R*, 6 R* )-B21 de l'exemple 1

Cette synthèse est illustrée par la Figure 2.

### (4 R *)-[(6 R *)-(8,12,12-triméthylcyclohex-7-èn-6-yl)((trimethyl silyl)oxy)methyl]dihydrofuran-2(3 H )-one (4 R *, 6 R *)-B45a

Une solution de (4*R**, 6*R**)-B21 (111.1 mg, 0.47 mmol) dans le TMS-imidazole (2.1 mL, 14.00 mmol, 30.0 équiv.) a été agitée pendant 1 h à 50 °C. Le mélange réactionnel a été refroidi à température ambiante et agité pendant 1 h. Le mélange a été dissous avec de l'éther de pétrole (5 mL), lavé avec de la saumure (2 x 5 mL), séché sur Na₂ SO₄ et les solvants ont été éliminés pour obtenir le produit brut (4*R**, 6*R**)-B45a de formule 15 avec R⁴ désignant un groupe méthyle (135,5 mg, quantitatif) sous la forme d'une huile incolore. Le produit brut a été utilisé sans purification dans l'étape suivante.

### (4R*, 6R*)-B45a

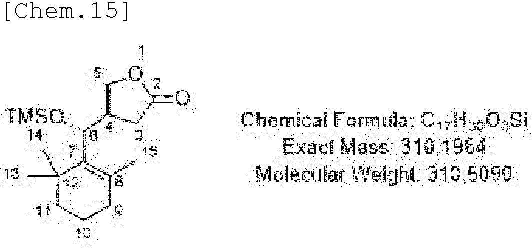

RMN ¹H (500 MHz, DMSO-d₆) δ 4.63-4.54 (1H, m, H-6), 4.33 (1H, t, *J* = 8.0 Hz,
H-5a), 4.17 (1H, t, *J* = 8.0 Hz, H-5b), 3.00-2.95 (1H, m, H-4), 2.45 (1H, dd, *J* = 17.0,
8.5, Hz, H-3a), 2.20-2.11 (1H, m, H-3b), 1.99 (2H, t, *J* = 6.5 Hz, H-9), 1.72 (3H, s, H-15), 1.61 (2H, quint, *J* = 6.5 Hz, H-10), 1.40-1.38 (2H, m, H-11), 1.13 (3H, s, H-13 ou H-14), 1.09 (3H, s, H-13 ou H-14), 0.10 (9H, s, H-TMS).

RMN ¹³C (125 MHz, DMSO-d₆) δ 175.8 (C-2), 137.6 (C-7), 130.7 (C-8), 71.5 (C-6), 70.2 (C-5), 41.6 (C-4), 41.0 (C-11), 33.2 (C-9), 31.3 (C-3), 28.8 (C-12), 28.4 (C-13 ou C-14), 28.3 (C-13 ou C-14), 20.2 (C-15), 18.0 (C-10), 0.32 (C-TMS).

IR (film) νₘₐₓ 2925, 2850, 1782, 1465, 1253, 1173, 1067, 883, 839, 747 cm⁻¹.

HRESIMS *m*/*z* 311.2036 [M + H]⁺ (calc. pour C₁₇H₃₁O₃Si, 311.2042).

### (4 R *)-3-( E )-(Hydroxyméthylène)-4-[(6 R *)-(8,12,12-triméthylcyclohex-7-èn -6-yl)((triméthylsilyl)oxy)méthyl]dihydrofuran-2(3 H )-one ((4 R *, 6 R *)-B46a)

À une solution de (4*R**, 6*R**)-B45a (13,0 mg, 0,04 mmol) dans du THF anhydre
(0,4 mL) à -40 °C sous argon, a été ajouté du formiate d'éthyle (32 µL, 0,40 mmol, 10,0 équiv.) et du tert-BuOK (33,3 mg, 0,28 mmol, 7,0 équiv.). Le mélange a été agité pendant 1 h à -40 °C, puis a été réchauffé à -10 °C et agité pendant 1 h supplémentaire. Le milieu réactionnel a été dilué avec de l'EtOAc (5 mL), lavée avec de l'eau (2 x 5 mL) et une solution aqueuse de NH₄Cl saturé (2 x 5 mL). La phase organique a été séchée sur Na₂SO₄et concentrée sous pression réduite pour obtenir le produit brut désiré (4R*, 6R*)-B46a de formule 16 avec R⁴ désignant un groupe méthyle (10.6 mg).

Le produit brut a été utilisé sans aucune purification dans l'étape suivante.

### (4R*, 6R*)-B46a

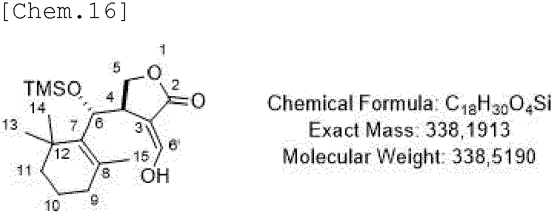

IR (film) νₘₐₓ 3464, 2925, 1763, 1462, 1379, 1253, 1219, 1178, 1067, 977, 839 cm⁻¹.

HRESIMS *m*/*z* 339.2001 [M + H]⁺ (calc. pour C₁₈H₃₁O₄Si, 339.1992).

### (±) -SdL781

A une solution de (*4R*, 6R**)-B46a (28,1 mg, 0,08 mmol) dans du THF anhydre (0,8 mL) à -78 °C sous argon, on a ajouté du *tert*-BuOK (14,1 mg, 0,12 mmol, 1,5 équiv.). A ce mélange a été additionné du 5-bromo-3-méthylfurane-2(5*H*)-one D4 [16] (21,2 mg, 0,12 mmol, 1,5 équiv.) dans du THF anhydre (0,8 mL). Le milieu réactionnel a été réchauffé à température ambiante et a été agité pendant une nuit. Le mélange réactionnel a été dissous dans EtOAc (5 mL), lavé avec de l'eau (2 x 5 mL) et de la saumure (2 x 5 mL) et séché sur Na₂SO₄. Les solvants ont été éliminés et le produit brut a été purifié par PTLC (éther de pétrole/EtOAc, 60:40) pour obtenir les produit (±)-SdL781 F1 de formule 22 (14,7 mg, 32% en 3 étapes) et (±)-SdL781 F2 de formule 23 (14,0 mg, 31% en 3 étapes). (±)-SdL781 F1 et et (±)-SdL781 F2 sont des analogues de la cannalactone de type silyle répondant à la formule générale 4 avec R⁴ désignant un groupe méthyle.

### (±)-SdL781 F1

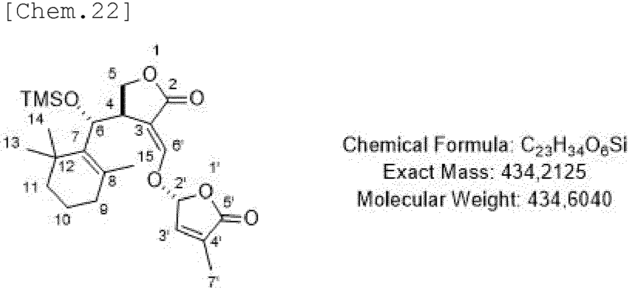

RMN ¹H (500 MHz, CDCl₃) δ 7.45 (1H, s, H-6'), 6.89 (1H, s, H-3'), 6.09 (1H, s, H-2'), 4.59-4.51 (2H, m, H-6 et H-5a), 4.13 (1H, t, J = 8.0 Hz, H-5b), 3.60-3.54 (1H, m, H-4), 2.00 (3H, s, H-7'), 1.88-1.81 (2H, m, H-9), 1.53-1.46 (2H, m, H-10), 1.33-1.27 (2H, m, H-11), 1.23 (3H, s, H-15), 1.08 (3H, s, H-13 ou H-14), 0.99 (3H, s, H-13 ou H-14), 0.05 (9H, s, H-TMS).

RMN ¹³C (125 MHz, CDCl₃) δ 172.4 (C-2), 170.2 (C-5'), 148.6 (C-6'), 142.0 (C-7), 140.9 (C-3'), 136.2 (C-4'), 136.2 (C-12), 100.7 (C-2'), 96.9 (C-3), 72.2 (C-6), 69.2
(C-5), 45.1 (C-4), 40.9 (C-9), 34.7 (C-11), 29.9 (C-15), 29.9 (C-8), 29.3 (C-13 et C-14), 19.1 (C-10), 11.9 (C-7'), 0.5 (C-TMS).

IR (film) νₘₐₓ 2954, 2920, 2853, 1784, 1755, 1686, 1462, 1342, 1254, 1191, 1082, 1026, 955, 887, 843, 752 cm⁻¹.

HRESIMS m/z 435.2189 [M + H]⁺ (calc. pour C₂₃H₃₅O₆Si, 435.2203).

### (±)-SdL781 F2

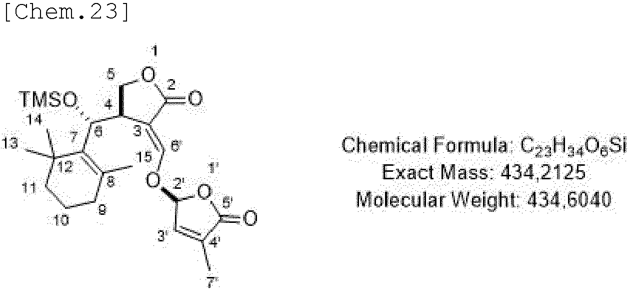

RMN ¹H (500 MHz, CDCl₃) δ 7.39 (1H, s, H-6'), 6.87 (1H, s, H-3'), 6.11 (1H, s,
H-2'), 4.61 (1H, d, J = 5.5 Hz, H-6), 4.56 (1H, d, J = 8.5 Hz, H-5a), 4.14 (1H, t, J = 8.5
Hz, H-5b), 3.58-3.53 (1H, m, H-4), 2.01 (3H, s, H-7'), 1.92 (2H, t, J = 7.0 Hz, H-9), 1.61-1.52 (2H, m, H-10), 1.42-1.34 (2H, m, H-11), 1.23 (9H, s, H-13, H-14 et H-15), 0.05 (9H, s, H-TMS).

RMN ¹³C (175 MHz, CDCl₃) δ 172.3 (C-2), 170.3 (C-5'), 149.6 (C-6'), 140.9 (C-7),
140.8 (C-3'), 136.4 (C-4'), 135.9 (C-12), 100.4 (C-2'), 96.2 (C-3), 72.0 (C-6), 69.0 (C-5), 45.1 (C-4), 41.0 (C-9), 34.7 (C-11), 29.9 (C-13 et C-14), 29.6 (C-8), 29.4 (C-15), 19.3 (C-10), 11.0 (C-7'), 0.4 (C-TMS).

IR (film) νₘₐₓ 2959, 2923, 2853, 1788, 1755, 1683, 1463, 1342, 1252, 1209, 1180, 1085, 1024, 958, 887, 842, 752 cm⁻1

HRESIMS m/z 435.2188 [M + H]⁺ (calc. pour C₂₃H₃₅O₆Si, 435.2203).

### EXEMPLE 5 : synthèse d'un analogue de la cannalactone de type alcool selon l'invention, à partir du diastéréoisomère (4 R*, 6 R* )-B21 de l'exemple 1

Cette synthèse est illustrée par la Figure 2.

### (4 R *)-[(6 R *)-{(Triéthylsilyl)oxy}(8,12,12-triméthylcyclohex-7-èn-6yl)méthyl]dihydrofuran-2(3 H )-one

### ((4 R *, 6 R *)-B45b)

À une solution de (4R*, 6R*)-B21 (12,4 mg, 0,05 mmol) dans la pyridine (0,4 mL), a été ajouté du 4-diméthylamino pyridine (DMAP) (1,9 mg, 2 µmol, 0,3 équiv.) et du chlorure de triéthylsilyle (TESCl) (50 µL, 0,30 mmol, 6,0 équiv.). Le mélange a été agité pendant 24 h. Le mélange réactionnel a été dissous avec CH₂Cl₂ (5 mL), lavé avec une solution aqueuse de NaHCO₃ saturé (2 x 5 mL) et séché sur Na₂SO₄. Les solvants ont été éliminés et le produit brut a été purifié par chromatographie sur
colonne de silice (éther de pétrole/EtOAc, 100:0 à 80:20 sur 10 min) pour obtenir le produit pur (4*R**, 6*R**)-B45b de formule 15 avec R⁴ désignant un groupe éthyle (12,3 mg, 70%) sous la forme de deux conformères comme une huile incolore.

### (4R*, 6R*)-B45b

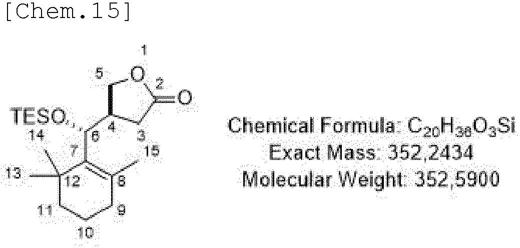

Conformère 1 :
RMN ¹H (500 MHz, DMSO-d6) δ 4.71 (1H, d, J = 11.0 Hz, H-6), 4.40 (1H, t, J =
   8.0 Hz, H-5a), 4.22 (1H, q, J = 4.5 Hz, H-5b), 3.06-3.00 (1H, m, H-4), 2.06 (2H, dd, J = 16.5, 6.5 Hz, H-3), 2.02-1.97 (2H, m, H-9), 1.68-1.64 (2H, m, H-10), 1.63 (3H, s, H-15), 1.43-1.37 (2H, m, H-11), 1.17 (3H, s, H-13 ou H-14), 1.10 (3H, s, H-13 ou H-14), 0.92 (9H, t, J = 7.0 Hz, H-CH3-TES), 0.58 (6H, q, J = 7.0 Hz, H-CH2-TES).
RMN ¹³C (125 MHz, DMSO-d6) δ 177.5 (C-2), 136.4 (C-7), 132.3 (C-8), 74.4 (C-6), 72.6 (C-5), 42.4 (C-4, C-11), 34.4 (C-12), 33.4 (C-9), 32.1 (C-3), 30.7 (C-13 ou C-14),
   30.6 (C-13 ou C-14), 21.0 (C-15), 18.9 (C-10), 7.16 (C-CH3-TES), 5.3 (C-CH2-TES).

Conformère 2 :
RMN ¹H (500 MHz, DMSO-d₆) δ 4.31 (2H, d, J = 5.5.0 Hz, H-5), 4.27 (1H, d, J = 8.5 Hz, H-6), 2.99-2.94 (1H, m, H-4), 2.29 (2H, d, J = 9.5 Hz, H-3), 1.96-1.85 (2H, m, H-9), 1.78 (3H, s, H-15), 1.59-1.51 (2H, m, H-10), 1.51-1.37 (1H, m, H-11a), 1.34-1.30 (1H, m, H-11b), 1.08 (3H, s, H-13 ou H-14), 0.90 (3H, s, H-13 ou H-14), 0.92 (9H, t, J = 7.0 Hz, H-CH₃-TES), 0.58 (6H, q, J = 7.0 Hz, H-CH₂-TES).
RMN ¹³C (125 MHz, DMSO-d₆) δ 177.2 (C-2), 137.0 (C-7), 133.6 (C-8), 71.5 (C-6), 71.2 (C-5), 44.1 (C-4), 40.6 (C-11), 34.7 (C-12), 34.6 (C-9), 33.1 (C-3), 30.2 (C-13 ou C-14), 29.2 (C-13 ou C-14), 21.9 (C-15), 19.4 (C-10), 7.16 (C-CH₃-TES), 5.5 (C-CH₂TES).

IR (film) νₘₐₓ 2963, 2928, 2881, 1782, 1666, 1460, 1412, 1371, 1241, 1175, 1069, 1006, 819, 741 cm⁻¹

HRESIMS *m*/*z* 353.2511 [M + H]⁺ (calc. pour C₂₀H₃₇O₃Si, 353.2512).

### (4R*, 6R*)-B48

A une solution de (4*R**, 6*R**)-B45b (21,5 mg, 0,06 mmol) dans du THF anhydre
(0,5 mL) à -40 °C sous argon, a été ajouté du formiate d'éthyle (50 µL, 0,60 mmol,
   10,0 équiv.) et du *tert*-BuOK (47,1 mg, 0,42 mmol, 7,0 équiv.). Le mélange a été
agité pendant 1 h à 0 °C puis a été refroidi à -78 °C. Ce mélange a été additionné de 5-bromo-3-méthylfurane-2(5*H*)-one D4 [16 (15,9 mg, 0,09 mmol, 1,5 équiv.) dans du THF anhydre (0,5 mL). Le milieu réactionnel a été réchauffé à température ambiante et a été agité pendant une nuit. Le mélange a été dissous dans EtOAc (5 mL), lavé avec de l'eau (2 x 5 mL) et de la saumure (2 x 5 mL) et séché sur Na₂SO₄. Les solvants ont été éliminés et le produit brut a été purifié par chromatographie sur colonne de gel de silice (heptane/EtOAc, 100:0 à 70:30) pour obtenir le produit (4*R**,6*R**)-B48 F1 de formule 24 (6.1 mg, 21%) et (4R*, 6R*)-B48 F2 de formule 25 (7.0 mg, 25%).

### (4R*, 6R*)-B48 F1

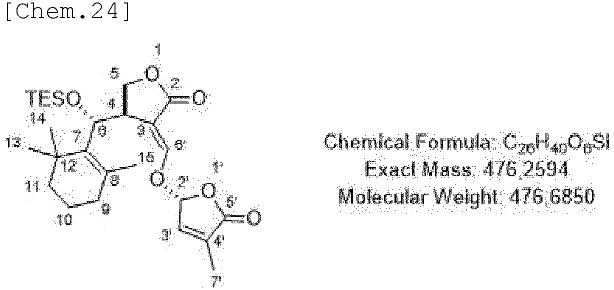

RMN ¹H (500 MHz, CDCl₃) δ 7.40 (1H, s, H-6'), 6.86 (1H, t, J = 1.5 Hz, H-3'), 6.11
(1H, s, H-2'), 4.64-4.59 (1H, m, H-5a), 4.58-4.54 (1H, m, H-6), 4.16 (1H, t, J = 8.5 Hz,
H-5b), 3.57-3.53 (1H, m, H-4), 2.01 (3H, t, J = 1.5 Hz, H-7'), 1.91 (2H, t, J = 6.5 Hz, H-9), 1.59-1.54 (2H, m, H-10), 1.39-1.35 (2H, m, H-11), 1.19 (3H, s, H-15), 1.06 (3H, s, H-13 ou H-14), 0.92 (3H, s, H-13 ou H-14), 0.90 (9H, t, J = 7.5 Hz, H-CH₃-TES), 0.55 (6H, q, J = 7.5 Hz, H-CH₂-TES).

RMN ¹³C(125 MHz, CDCl₃) δ 172.3 (C-2), 170.3 (C-5'), 149.9 (C-6'), 140.8 (C-3'),
136.6 (C-12), 136.4 (C-4'), 133.4 (C-7), 102.0 (C-3), 100.5 (C-2'), 71.9 (C-6), 69.0
(C-5), 45.3 (C-4), 40.8 (C-9), 34.9 (C-11), 32.1 (C-8), 30.1 (C-13 ou C-14), 29.9 (C-15), 29.5 (C-13 ou C-14), 19.3 (C-10), 11.0 (C-7'), 7.1 (C-CH₃-TES), 5.3 (C-CH₂TES).

IR (film) νₘₐₓ 2963, 2922, 1787, 1757, 1681, 1466, 1343, 1259, 1184, 1084, 1018, 951, 862, 800,

743 cm⁻¹.

HRESIMS *m*/*z* 477.2659 [M + H]⁺ (calc. pour C₂₆H₄₁O₆Si, 477.2672).

### (4R*, 6R*)-B48 F2

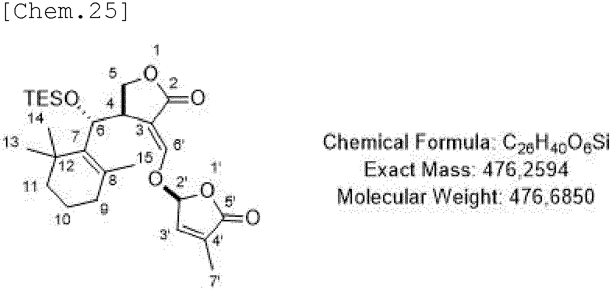

RMN ¹H (500 MHz, CDCl₃) δ 7.46 (1H, s, H-6'), 6.88 (1H, t, J = 1.5 Hz, H-3'), 6.09
(1H, s, H-2'), 4.62-4.58 (1H, m, H-5a), 4.88 (1H, s, H-6), 4.17-4.13 (1H, m, H-5b),
3.59-3.54 (1H, m, H-4), 2.01 (3H, s, H-7'), 1.88-1.83 (2H, m, H-9), 1.52-1.45 (2H, m,
H-10), 1.32-1.323 (2H, m, H-11), 1.23 (3H, s, H-15), 1.02 (3H, s, H-13 ou H-14), 0.93 (3H, s, H-13 ou H-14), 0.90 (9H, t, J = 7.0 Hz, H-CH₃-TES), 0.54 (6H, q, J = 7.5 Hz, H-CH₂-TES).

RMN ¹³C (125 MHz, CDCl₃) δ 172.3 (C-2), 170.2 (C-5'), 150.5 (C-6'), 140.8 (C-3'),
136.6 (C-12), 136.2 (C-4'), 133.5 (C-7), 100.6 (C-2'), 100.1 (C-3), 71.8 (C-6), 69.3
(C-5), 45.3 (C-4), 40.7 (C-9), 34.8 (C-11), 32.1 (C-8), 29.9 (C-13 ou C-14), 29.9 (C-15), 29.5 (C-13 ou C-14), 19.2 (C-10), 10.9 (C-7'), 7.1 (C-CH₃-TES), 5.3 (C-CH₂TES).

IR (film) νₘₐₓ 2960, 2922, 1788, 1753, 1679, 1460, 1259, 1184, 1091, 1015, 868, 797 cm⁻¹.

HRESIMS *m*/*z* 477.2564 [M + H]⁺ (calc. pour C₂₆H₄₁O₆Si, 477.2672).

### (±)-SdL628 F1

### Méthode 1

À une solution de (±)-SdL781 F1 (14,3 mg, 0,032 mmol) dans CH₃CN (0,2 mL) et de l'eau (2 gouttes) a été ajouté une solution de Sc(OTf)₃ (0,1 mg, 16 µmol, 0,5 mol%) dans CH₃CN (0,2 mL). Le mélange résultant a été agité pendant 1 h 30 à température ambiante et hydrolysé avec un tampon phosphate aqueux (2 mL, pH 7). La phase organique a été extraite avec CH₂Cl₂ (3 x 2 mL), et les extraits combinés ont été lavés avec de la saumure (2 x 3 mL), puis séchés sur Na₂SO₄. Les solvants ont été éliminés et le produit brut a été purifié par PTLC (éther de pétrole/EtOAc, 60:40) pour obtenir le produit (±)-SdL628 F1 de formule 26 (2,4 mg, 22%) sous forme d'huile incolore.

### Méthode 2

A une solution de (4*R**, 6*R**)-B48 F1 (6,1 mg, 0,01 mmol) dans du THF anhydre (1 mL) sous argon a été ajoutée une solution de 3HF.NEt₃ (20 µL, 0,13 mmol, 10,0 équiv.). Le mélange a été agité pendant une nuit à 50 °C. La phase organique a été refroidie avec une solution aqueuse de NaHCO₃ saturé (1 mL) et extraite avec de l'EtOAc (3 x 2 mL). La phase organique a été séchée sur Na₂SO₄ et les solvants ont été éliminés. Le produit brut a été purifié par PTLC (heptane/EtOAc, 60:40) pour obtenir le produit (±)-SdL628 F1 de formule 26 (3,9 mg, 83%) sous forme d'huile incolore.

### (±)-SdL628 F1

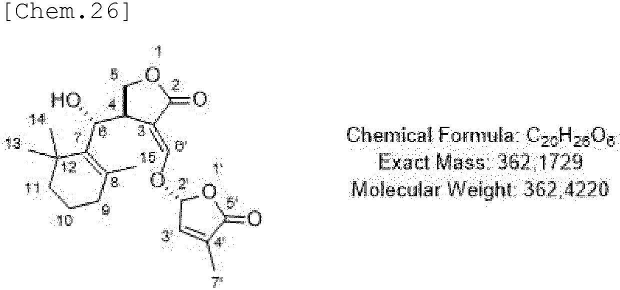

RMN ¹H (500 MHz, CDCl3) δ 7.35 (1H, s, H-6'), 6.82 (1H, s, H-3'), 6.06 (1H, s, H-2'), 4.60 (1H, d, J = 10.0 Hz, H-6), 4.23 (1H, dd, J = 9.0, 6.0 Hz, H-5a), 4.17 (1H, d, J = 10.0 Hz, H-5b), 3.86-3.82 (1H, m, H-4), 1.83 (3H, s, H-7'), 1.82-178 (2H, m, H-9), 1.43-1.40 (2H, m, H-10), 1.39-1.34 (2H, m, H-11), 1.23 (3H, s, H-15), 1.06 (3H, s, H-13 ou H-14), 0.83 (3H, s, H-13 ou H-14).

RMN ¹³C (175 MHz, CDCl3) δ 172.2 (C-2), 170.2 (C-5'), 150.8 (C-6'), 140.7 (C-3'),
138.1 (C-7), 136.6 (C-12), 134.7 (C-4'), 109.9 (C-3), 100.4 (C-2'), 71.0 (C-5), 70.7 (C-6), 43.4 (C-4), 40.2 (C-9), 34.9 (C-8), 34.8 (C-11), 29.9 (C-15), 29.2 (C-13 ou C-14), 29.1 (C-13 ou C-14), 21.7 (C-7'), 19.5 (C-10).

IR (film) νmax 3479, 2930, 2861, 1785, 1754, 1682, 1457, 1346, 1191, 1085, 1023, 958 cm⁻¹.

HRESIMS *m*/*z* 363.1808 [M + H]⁺ (calc. pour C20H27O6, 363.1807).

### (±)-SdL628 F2

### Méthode 1

A une solution de (±)-SdL781 F2 (19,2 mg, 0,044 mmol) dans CH₃CN (0,3 mL) et de l'eau (3 gouttes), a été ajoutée une solution de Sc(OTf)₃ (0,1 mg, 22 µmol, 0,5 mol%) dans CH₃CN (0,3 mL). La phase organique a été extraite avec CH₂Cl₂ (3 x 2 mL), lavée avec de la saumure (2 x 3 mL) et séchée sur Na₂SO₄. Les solvants ont été éliminés et le produit brut a été purifié par PTLC (éther de pétrole/EtOAc, 60:40) pour obtenir le produit (±)-SdL628 F2 de formule 27 (3,8 mg, 19 %) sous forme d'huile incolore.

### Méthode 2

A une solution de (4*R**, 6*R**)-B48 F2 (7,0 mg, 0,02 mmol) dans du THF anhydre (1 mL) sous argon, a été ajoutée une solution de 3HF.NEt₃ (20 µL, 0,18 mmol, 10,0 équiv.). Le mélange a été agité pendant une nuit à 50 °C. La phase organique a été
refroidie avec une solution aqueuse de NaHCO₃ saturé (1 mL) et extraite avec de l'EtOAc (3 x 2 mL). La phase organique a été séchée sur Na₂SO₄ et les solvants ont été éliminés. Le produit brut a été purifié par PTLC. brut a été purifié par PTLC (heptane/EtOAc, 60:40) pour obtenir le produit (±)-SdL628 F2 de formule 27 (6,3 mg, quantitatif) sous forme d'huile incolore.

### (±)-SdL628 F2

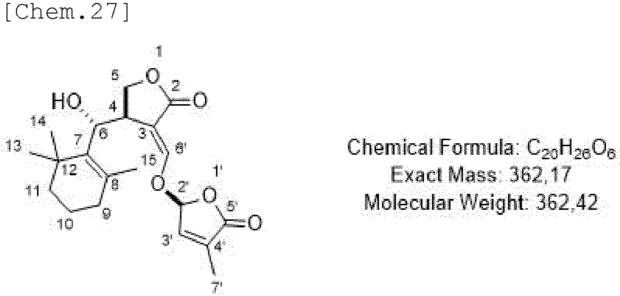

RMN ¹H (500 MHz, CDCl3) δ 7.46 (1H, s, H-6'), 6.87 (1H, s, H-3'), 6.04 (1H, s, H-2'), 4.59 (1H, d, J = 10.5 Hz, H-6), 4.22 (1H, dd, J = 8.5, 6.5 Hz, H-5a), 4.16 (1H, d, J = 10.5 Hz, H-5b) , 3.86-3.82 (1H, m, H-4), 1.89-1.82 (1H, m, H-9a), 1.79 (3H, s,
H-7'), 1.59-1.54 (1H, m, H-9b), 1.41-1.36 (2H, m, H-10), 1.31-1.25 (2H, m, H-11),
1.23 (3H, s, H-15), 1.03 (3H, s, H-13 ou H-14), 0.83 (3H, s, H-13 ou H-14)

RMN ¹³C (175 MHz, CDCl₃) δ 172.2 (C-2), 170.2 (C-5'), 152.1 (C-6'), 140.7 (C-3'),
138.0 (C-7), 136.1 (C-12), 134.7 (C-4'), 109.3 (C-3), 100.9 (C-2'), 71.1 (C-5), 70.7 (C-6), 43.3 (C-4), 39.9 (C-9), 34.8 (C-8), 34.5 (C-11), 29.9 (C-15), 29.2 (C-13 ou C-14), 28.9 (C-13 ou C-14), 21.6 (C-7'), 19.1 (C-10).

IR (film) νₘₐₓ 3479, 2961, 2925, 2861, 1783, 1750, 1682, 1345, 1260, 1189, 1089, 1022, 955, 801 cm⁻¹.

HRESIMS *m*/*z* 363.1793 [M + H]⁺ (calc. pour C₂₀H₂₇O₆, 363.1807).

(±)-SdL628 F1 et et (±)-SdL628 F2 sont des analogues de la cannalactone de type alcool répondant à la formule 5 générale.

### EXEMPLE 6 : évaluation de l'activité biologique

L'évaluation biologique des analogues de la cannalactone selon l'invention synthétisés aux exemples 2 (analogues de type aromatique SdL625 F1 + SdL625 F2), 3 (analogues de type diène SdL646 F1 + SdL646 F2), 4 (analogues de type silylé SdL781F1 + SdL781F2) et 5 (analogues de type alcool SdL628 F1 + SdL628 F2) a été testée pour l'une des activités que ces analogues pourraient assurer dans le chanvre : la germination d'une plante parasite, *P. ramosa.*

Les résultats ont été comparés avec ceux obtenus sur un analogue synthétique de référence, le (±)-GR24 [17], [18] et à la (+)-cannalactone naturelle [1], [4] isolée des exsudats de chanvre :

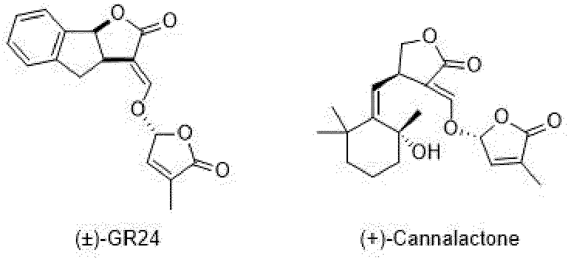

En particulier, l'activité de stimulation de la germination de graines de plantes parasites a été évaluée sur deux populations de *P. ramosa. P. ramosa 1,* prélevée sur colza alors que P. *ramosa* 2a est récoltée sur une parcelle de chanvre [2]- [4] .

### Protocole

Le protocole utilisé, développé par Pouvreau et al. [19], permet de tester l'activité biologique de molécules ou d'extraits biologiques sur la germination de graines de plantes parasites dans des plaques de 96 puits en routine (comme illustré par la [Fig.3]). Cette technique s'affranchit du comptage de graines germées qui était utilisé jusque-là.

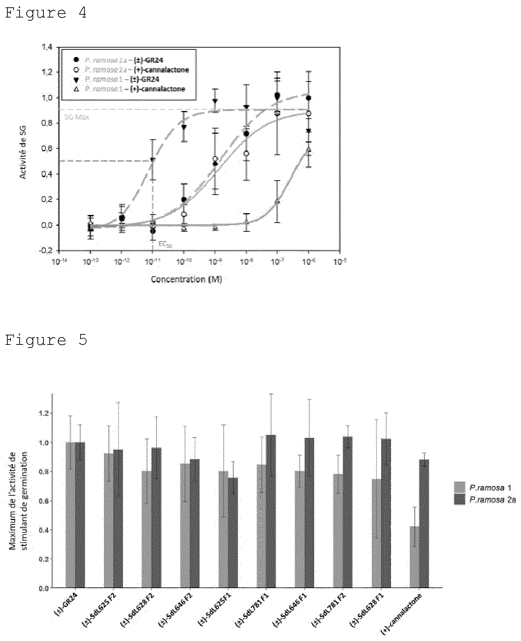

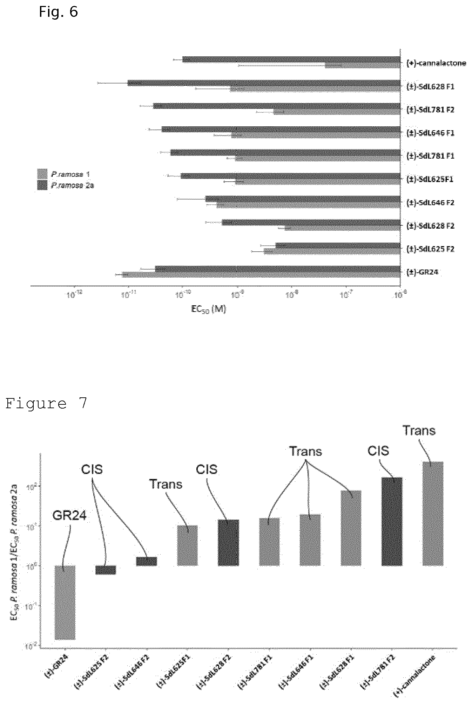

Les analogues de la cannalactone synthétisés aux exemples 2 à 5 ont été testés par
ce protocole et leur activité maximale (Germination maximale) (comme illustré par la [Fig.4]) ainsi que la concentration efficace médiane (EC₅₀) (comme illustré par la [Fig.6]) ont été modélisées à partir de la courbe dose-réponse (comme illustré par la [Fig.5]). Cette mesure représente la concentration effective qui induit une réponse médiane entre la ligne de base et l'effet de germination maximum. La [Fig.5] montre que les analogues des exemples 2 à 5 semblent tous démontrer une capacité de germination maximale sur les deux populations de type 2a du même ordre que le (±)-GR24. Leur capacité de germination maximale sur le type 1 semble réduite, la tendance observée se rapproche davantage de celle obtenue pour la (+)-cannalactone.

Tous les analogues synthétisés dans les exemples 2 à 5 présentent des EC₅₀
inférieures à 10⁻⁸ M pour les deux types de populations ([Fig.6]). Certaines molécules comme (±)-SdL628 F1 et (±)-SdL781 F2 possèdent une activité biologique sur le P. *ramosa* 2a à plus faible concentration donc supérieure à celle de la molécule naturelle
   (EC₅₀ [(±)-SdL628 F1] = 9,6 × 10⁻¹² M versus EC₅₀ [(+)-cannalactone] = 1,0 × 10⁻¹⁰
M). La spécificité de ces analogues est également accentuée (comme illustré par la [Fig.7]), r_{EC50}[(±)-SdL628 F1] ~ 100 versus r_{EC50} [(+)-SdL19] ~ 10, et se rapproche de celle de la cannalactone naturelle.

### Références bibliographiques

1. Hamzaoui, O. et al., Proceedings of the 15th World Congress on Parasitic Plants; Amsterdam, The Netherlands (2019): 32.
2. Stojanova, B., Delourme, R., Duffé, P., Delavault, P. & Simier, P. Genetic differentiation and host preference reveal non-exclusive host races in the generalist parasitic weed Phelipanche ramosa. Weed Res. 59, 107-118, doi:10.1111/wre.12353 (2019).
3. Huet, S., Pouvreau, J.-B., Delage, E., Delgrange, S., Marais, C., Bahut, M., Delavault, P., Simier, P. & Poulin, L. Populations of the Parasitic Plant Phelipanche ramosa Influence Their Seed Microbiota. Front. Plant Sci. 11, 1075, doi:10.3389/fpls.2020.01075 (2020).
4. Daignan Fornier, S., de Saint Germain, A., Retailleau, P., Pillot, J.-P., Taulera, Q., Andna, L., Miesch, L., Rochange, S., Pouvreau, J.-B. & Boyer, F.-D. Noncanonical Strigolactone Analogues Highlight Selectivity for Stimulating Germination in Two Phelipanche ramosa Populations. J. Nat. Prod. 85, 1976-1992, doi:10.1021/acs.jnatprod.2c00282 (2022).
5. Delavault, P., Montiel, G., Brun, G., Pouvreau, J. B., Thoiron, S. & Simier, P. Communication Between Host Plants and Parasitic Plants. Adv. Bot. Res. 82, 55-82, doi:10.1016/bs.abr.2016.10.006 (2017).
6. Xie, X., Yoneyama, K. & Yoneyama, K. The Strigolactone Story. Annu. Rev. Phytopathol. 48, 93-117, doi:10.1146/annurev-phyto-073009-114453 (2010).
7. Daignan-Fornier, S.; Keita, A.; Boyer, F.-D., Chemistry of Strigolactones, Key Players in Plant Communication. ChemBioChem, n/a, (n/a), doi:10.1002/ cbic.202400133 (2024).
8. Cook, C. E., Whichard, L. P., Turner, B. & Wall, M. E. Germination of Witchweed (Striga Lutea Lour) - Isolation and Properties of a Potent Stimulant. Science 154, 1189-1190, doi:10.1126/science.154.3753.1189 (1966).
9. Akiyama, K., Matsuzaki, K. & Hayashi, H. Plant sesquiterpenes induce hyphal branching in arbuscular mycorrhizal fungi. Nature 435, 824-827, doi:10.1038/nature03608 (2005).
10. Gomez-Roldan, V., Fermas, S., Brewer, P. B., Puech-Pages, V., Dun, E. A., Pillot, J.-P., Letisse, F., Matusova, R., Danoun, S., Portais, J.-C., Bouwmeester, H., Bécard, G., Beveridge, C. A., Rameau, C. & Rochange, S. F. Strigolactone inhibition of shoot branching. Nature 455, 189-194, doi:10.1038/nature07271 (2008).
11. Umehara, M., Hanada, A., Yoshida, S., Akiyama, K., Arite, T., TakedaKamiya, N., Magome, H., Kamiya, Y., Shirasu, K., Yoneyama, K., Kyozuka, J. & Yamaguchi, S. Inhibition of shoot branching by new terpenoid plant hormones. Nature 455, 195-200, doi:10.1038/nature07272 (2008).
12. Lopez-Obando, M., Ligerot, Y., Bonhomme, S., Boyer, F.-D. & Rameau, C. Strigolactone biosynthesis and signaling in plant development. Development 142, 3615-3619, doi:10.1242/dev.120006 (2015).
13. Boyer, F.-D., de Saint Germain, A., Pillot, J. P., Pouvreau, J.-B., Chen, V. X., Ramos, S., Stevenin, A., Simier, P., Delavault, P., Beau, J.-M. & Rameau, C. Structure-activity relationship studies of strigolactone-related molecules for branching inhibition in garden pea: molecule design for shoot branching. Plant Physiol. 159, 1524-1544, doi:10.1104/pp.112.195826 (2012).
14. Boyer, F.-D., de Saint Germain, A., Pouvreau, J.-B., Clavé, G., Pillot, J.-P., Roux, A., Rasmussen, A., Depuydt, S., Lauressergues, D., Frei dit Frey, N., Heugebaert, T. S. A., Stevens, C. V., Geelen, D., Goormachtig, S. & Rameau, C. New Strigolactone Analogs as Plant Hormones with Low Activities in the Rhizosphere. Mol. Plant 7, 675-690, doi:10.1093/mp/sst163 (2014).
15. Jas, G. Ein einfacher Zugang zu 4-Brom-2-(tert-butyldimethylsiloxy)f uran aus Tetrahydro-2,4-dioxofuran. Synthesis 1991, 965-966, doi:10.1055/ s-1991-26618 (1991).
16. Macalpine, G. A.; Raphael, R. A.; Shaw, A.; Taylor, A. W.; Wild, H. J. Synthesis of Germination Stimulant (±)-Strigol. J. Chem. Soc., Perkin Trans. 1 1976, (4), 410-416. DOI: 10.1039/P19760000410.
17. de Saint Germain, A., Retailleau, P., Norsikian, S., Servajean, V., Pelissier, F., Steinmetz, V., Pillot, J.-P., Rochange, S., Pouvreau, J.-B. & Boyer, F.D. Contalactone, a contaminant formed during chemical synthesis of the strigolactone reference GR24 is also a strigolactone mimic. Phytochemistry 168, 112112, doi:10.1016/j.phytochem.2019.112112 (2019).
18. Johnson, A. W., Gowda, G., Hassanali, A., Knox, J., Monaco, S., Razavi, Z. & Rosebery, G. The Preparation of Synthetic Analogs of Strigol. J. Chem. Soc., Perkin Trans. 1, 1734-1743, doi:10.1039/P19810001734 (1981).
19. Pouvreau, J.-B.; Gaudin, Z.; Auger, B.; Lechat, M. M.; Gauthier, M.; Delavault, P.; Simier, P. A high-throughput seed germination assay for root parasitic plants. Plant Methods 9 (1), 32. doi: 10.1186/1746-4811-9-32 (2013).

## Revendications

1. Analogue de la cannalactone, **caractérisée en ce qu'**il répond à la formule générale (1) : 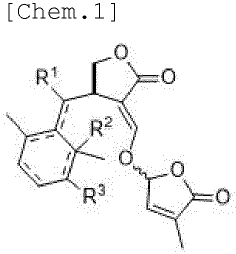 dans laquelle :
- R¹ désigne l'atome d'hydrogène H, le groupe hydroxyle OH ou le groupe OSiR⁴₃,
- R² et R³désignent chacun l'atome d'hydrogène H ou le radical méthyle CH₃,
- R⁴ désigne un groupement alkyle, et
- le cycle carboné à 6 chaînons pouvant être aromatique ou de typecyclohexène ou cyclohexane.

2. Analogue de la cannalactone selon la revendication 1, **caractérisé en ce qu'**il est aromatique de stéréochimie « *cis* » et « *trans »* et répond à la formule (2) : 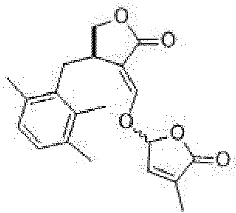 dans laquelle :
- R¹, R² et R³ désignent l'atome d'hydrogène H, et - le cycle carboné à 6 chaînons est aromatique.

3. Analogue de la cannalactone selon la revendication 1, **caractérisé en ce qu'**il est de type diène de stéréochimie « *cis* » et « *trans* » et répond à la formule (3) : 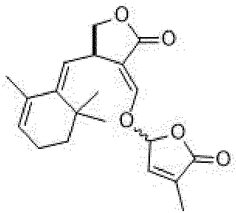 dans laquelle :
- R¹ et R³ désignent l'atome d'hydrogène H,
- R² désigne le groupement méthyle, et
- le cycle carboné à 6 chaînons est de type cyclohexène.

4. Analogue de la cannalactone selon la revendication 1, **caractérisé en ce qu'**il est de type syllilé de stéréochimie « *cis* » et « *trans* » et répond à la formule (4) : 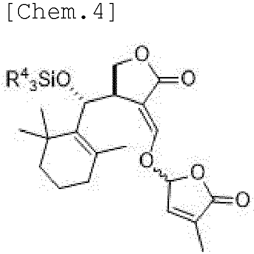 dans laquelle :
- R¹ désigne le groupe OSiR⁴₃,
- R² désigne le groupement méthyle, - R³ désigne l'atome d'hydrogène H, et
- le cycle carboné à 6 chaînons est de type cyclohexène.

5. Analogue de la cannalactone selon la revendication 1, **caractérisé en ce qu'**il est de type alcool de stéréochimie « *cis* » et « *trans* » et répond à la formule (5) : 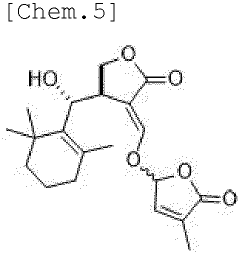 dans laquelle :
- R¹ désigne le groupe hydroxyle OH, et
- R² désigne le groupement méthyle, - R³ désigne l'atome d'hydrogène H, et
- le cycle carboné à 6 chaînons est de type cyclohexène.

6. Procédé de synthèse d'un analogue de la cannalactone tel que défini selon la revendication 2, **caractérisé en ce qu'**il comprend les étapes suivantes :
- une réaction A) de couplage du β-cyclocitral commercial avec unbromofurane C4 de formule (6) : 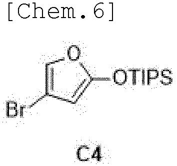 pour obtenir un alcool B20 de formule (7) : 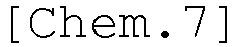 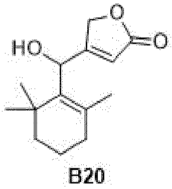
- une étape B) de réduction de l'alcool B20 de formule (7), pourobtenir un mélange de diastéréoisomères de l'alcool allylique, suivie une étape de séparation desdits diastéréoisomères pour retenir le diastéréoisomère (*4R*, 6R**)-B21 de formule (8) 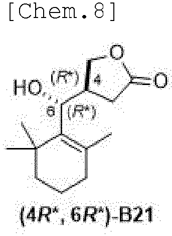
- une étape C2) d'époxydation du diastéréoisomère (4R*,6R*)-B21 de formule (8) pour obtenir un époxyalcool B22 de formule (9) 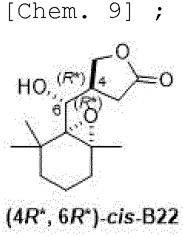
- une étape D2) de déshydratation et réarrangement de l'époxyalcoolB22 de formule (9) pour obtenir un composé benzylique B38 de formule (10) : 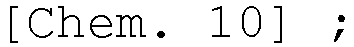 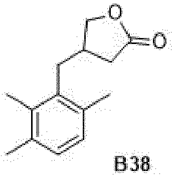
- une étape E2) de formylation en milieu basique du composébenzylique B38 de formule (10) pour obtenir un énol B40 de formule (11) : 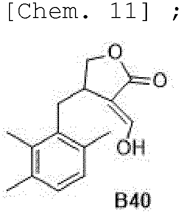
- un étape F2) d'O-alkylation de l'énol B40 pour obtenir l'analogue de formule (2).

7. Procédé de synthèse d'un analogue de la cannalactone tel que défini selon la revendication 3, **caractérisé en ce qu'**il comprend les étapes suivantes :
- les étapes A et B telles que définies dans la revendication 6, suiviespar
- une étape C3) de mésylation du diastéréoisomère (4*R**, 6*R**)-B21 de formule (8) pour obtenir après déshydratation et réarrangement le diène (*E*)-B25 de formule (13) 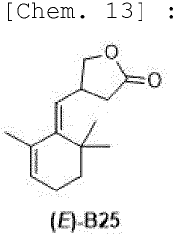
- une étape E3) de formylation en milieu basique du diène (*E*)-B25 de formule 13 pour obtenir un énol B42 de formule (14) : [Chem. 14] : 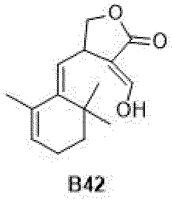
- un étape F3) d'*O*-alkylation de l'énol B42 pour obtenir l'analogue de formule (3).

8. Procédé de synthèse d'un analogue de la cannalactone tel que défini selon la revendication 4, **caractérisé en ce qu'**il comprend les étapes suivantes :
- les étapes A et B telles que définies dans la revendication 6, suiviesde
- une étape C4) de protection du diastéréoisomère (*4R*, 6R**)-B21 de formule 8 pour obtenir le composé protégé (4*R**, 6*R**)-B45 de formule (15) : [Chem. 15] : 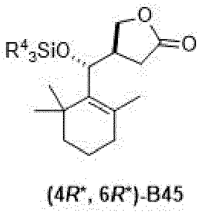
- une étape E4) de formylation en milieu basique du composé protégéde formule 16 pour obtenir un énol (4*R**, 6*R**)-B46 de formule (16) : 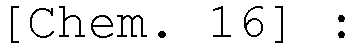 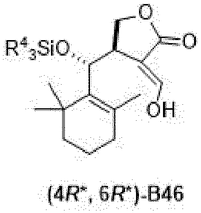
- un étape F4) d'*O*-alkylation de l'énol B46 pour obtenir l'analogue de formule (4).

9. Procédé de synthèse d'un analogue de la cannalactone tel que défini selon la revendication 6, **caractérisé en ce qu'**il comprend les étapes suivantes :
- la formation d'un analogue de la cannalactone tel que défini selonla revendication 4 conformément au procédé tel que défini selon la revendication 8, suivie par
- une étape G5) de déprotection et de séparation desdiastéréoisomères de l'analogue de formule (4), pour obtenir l'analogue de formule (5).

10. Utilisation d'un analogue de la cannalactone telle que définie selon l'une quelconque des revendications 1 à 5 ou telle qu'obtenue selon l'un des procédés de synthèse objet des revendications 6 à 9, en tant que stimulant de germination de graines de plantes parasites.

11. Utilisation selon la revendication 10, en tant que stimulant de germination des graines de *P*. *ramosa 1* et *P. ramosa* 2a.

12. Utilisation selon la revendication 10, pour la germination suicide de plantes parasites de type *Striga, Orobanche* et *Phelipanche.*
